(19) Europäisches Patentamt
European Patent Office
Office européen des brevets

(11) **EP 2 459 160 B1**

(12) **FASCICULE DE BREVET EUROPEEN**

(45) Date de publication et mention de la délivrance du brevet:
**03.09.2014 Bulletin 2014/36**

(21) Numéro de dépôt: **10752076.9**

(22) Date de dépôt: **22.07.2010**

(51) Int Cl.:
*A61K 8/44* *(2006.01)* *A61Q 19/06* *(2006.01)*
*A61Q 19/08* *(2006.01)*

(86) Numéro de dépôt international:
**PCT/FR2010/051550**

(87) Numéro de publication internationale:
**WO 2011/015758 (10.02.2011 Gazette 2011/06)**

(54) **UTILISATION DE N-HEXADECANOYL ISOLEUCINE COMME AGENT "VOLUMATEUR" ET/OU COMME AGENT "REPULPANT" DE LA PEAU HUMAINE**

VERWENDUNG VON ISOLEUKIN-N-HEXADEKANOYL ALS VOLUMISIERUNGS- ODER AUFPOLSTERUNGSMITTEL FÜR DIE MENSCHLICHE HAUT

USE OF ISOLEUCINE N-HEXADECANOYL AS A "VOLUMIZING" AND/OR "PLUMPING" AGENT FOR HUMAN SKIN

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **28.07.2009 FR 0955289**

(43) Date de publication de la demande:
**06.06.2012 Bulletin 2012/23**

(73) Titulaire: **Société d'Exploitation de Produits pour les Industries Chimiques SEPPIC 75007 Paris (FR)**

(72) Inventeurs:
• **CATTUZZATO, Laetitia F-81100 Castres (FR)**
• **CHEVROT, Nathalie F-75015 Paris (FR)**
• **DUMONT, Sandy F-81200 Caucalieres (FR)**
• **STOLTZ, Corinne F-94320 Thiais (FR)**

(74) Mandataire: **Conan, Philippe Claude L'Air Liquide SA Direction de la Propriété Intellectuelle 75, quai d'Orsay 75321 Paris Cedex 07 (FR)**

(56) Documents cités:
**WO-A1-90/14429     WO-A1-98/09611
FR-A1- 2 873 575**

• **MORELLE J: "LIPOAMINOACIDES ET COSMETOLOGIE", PARFUMS COSMETIQUES SAVONS DE FRANCE, PARIS, FR, vol. 3, no. 2, 1 février 1973 (1973-02-01) , pages 82-93, XP000106572,**

EP 2 459 160 B1

**Description**

La présente invention se rapporte au domaine des principes actifs cosmétiques et pharmaceutiques ainsi qu'aux compositions topiques en comportant.

L'augmentation de l'espérance de vie dans les pays développés s'est constamment accompagnée par un désir de "rajeunissement" qui s'est accentué depuis une trentaine d'années. Cette volonté se traduit par des tentatives pour retarder le phénomène de relâchement cutané des adultes vieillissant, qui se traduit notamment au niveau du visage par une modification des traits et des expressions des individus. Dans un même esprit de conserver une allure dynamique, « jeune », une silhouette galbée, traduisant de façon apparente le bien-être, certaines femmes sont également à la recherche de solutions destinées à raffermir la poitrine voire à en augmenter son volume. Cette évolution comportementale s'est accompagnée par un développement de la recherche scientifique dédiée à la peau ainsi que depuis une dizaine d'années par une croissance rapide du marché des produits cosmétiques dits "anti-âge" ou destinés à améliorer l'état général de la peau.

La présente invention s'inscrit dans ce cadre de la recherche de nouvelles molécules ou compositions qui, appliquées sur la peau produisent par leur activité propre, un effet « repulpant » de la peau et/ou « densifiant » de la peau et/ou « volumateur » de la peau et/ou améliorant l'élasticité de la peau de différentes parties du corps humain telles que les mains, les fesses, le visage, le buste féminin, les paupières.

Chez les mammifères, le tissu adipeux constitue une couche de graisse de réserve qui enveloppe le corps, mais dont l'épaisseur diffère selon la zone du corps, l'âge et le sexe. Chez la femme, le tissu adipeux représente de 20 à 25% de la masse totale, alors que chez l'homme il varie entre 15 et 20% de la masse totale. L'homme et la femme possèdent des sites de localisation différents du tissu adipeux, ceci étant du principalement aux différents facteurs hormonaux synthétisés par le corps humain selon le sexe. Du fait de son importance, le tissu adipeux participe à l'aspect général de la silhouette et du visage des êtres humains. On trouve généralement le tissu adipeux au niveau du visage, des membres, de l'abdomen, des mains, des fesses. Chez la femme, le tissu adipeux est également présent au niveau du buste, et plus particulièrement au niveau des seins. Le sein de la femme est constitué de la glande mammaire, du tissu conjonctif et du tissu adipeux. Le tissu adipeux représente une grande partie du volume des seins, et par conséquent sa proportion et sa répartition déterminent la taille et la forme du sein. Selon les étapes de la vie (grossesse, allaitement, jeunesse, amaigrissement prononcé, maladie), la proportion et la répartition du tissu adipeux du sein subissent des variations qui génèrent des modifications du volume et de la forme du sein, pouvant ainsi entrainer soit un relâchement soit un raffermissement du sein. Au niveau du visage le tissu adipeux permet de dessiner les traits et les expressions de chaque individu. Un amaigrissement trop important ou le vieillissement cutané peuvent induire une diminution de la proportion du tissu adipeux du visage, et entrainer une modification des traits et de l'expression des individus.

Le mécanisme du vieillissement de la peau peut être expliqué comme suit :

La peau est composée de trois tissus majoritaires avec, par ordre depuis la surface externe : l'épiderme, le derme et l'hypoderme.

- L'épiderme, est essentiellement composé de kératinocytes. Ces cellules prolifèrent dans l'assise basale puis se différencient progressivement pour donner les différentes couches de l'épiderme en migrant depuis la profondeur vers la surface, où elles desquament.
- Le derme est un tissu conjonctif dense et fibro-élastique, dont la production et le remodelage éventuel sont essentiellement assurés par les fibroblastes et qui est constitué de la matrice extracellulaire (MEC) composée de molécules fibreuses (collagéniques et élastiques), responsables des qualités esthétiques de la peau, ainsi que de la substance fondamentale. Les collagènes représentent 98% du poids sec du derme. La substance fondamentale, est composée de macromolécules qui comblent les espaces entre les cellules et les fibres ; de glycoprotéines de structure, qui permettent la connexion des cellules à la MEC et qui sont essentiellement localisées sous la jonction dermo-épidermique ; de protéoglycanes constitué s d e glycosaminoglycanes (GAG) liés à une protéine (liaison covalente).
- L'hypoderme constitue la couche profonde de la peau. Il est composé du tissu adipeux et du tissu conjonctif, et il est richement vascularisé et innervé. A l'interface du derme et des structures mobiles sous-jacentes (muscles, tendons, organes), l'hypoderme joue un rôle d'amortisseur et protège ces structures mobiles sous-jacentes vis-à-vis des pressions mécaniques extérieures. L'hypoderme, du fait de la nature du tissu adipeux, possède également un rôle énergétique, métabolique et thermique important. Il stocke notamment les apports énergétiques alimentaires en excès, sous forme de triglycérides (TG), phénomène connu sous le nom de lipogenèse, pour pouvoir ensuite les redistribuer en fonction des besoins du corps humain, phénomène connu sous le nom de lipolyse. Les principaux acteurs de ce tissu adipeux sont les adipocytes et leurs précurseurs, les pré-adipocytes. L'adipocyte se présente sous la forme d'une cellule ronde, extensible, possédant une grande vacuole lipidique prenant tout l'espace cytoplasmique et rejetant le noyau à la périphérie cellulaire. Le pré-adipocyte est une petite cellule de morphotype fibroblastique, ne possédant aucune activité métabolique liée au stockage d'énergie. Lorsqu'elle se trouve en présence de signaux extracellulaires adéquats, elle entame un processus de différenciation afin d'acquérir le phénotype d'adipocyte mature. Ce processus, largement décrit in vitro, prend plusieurs semaines et est rendu possible par l'expression coordonnées de différents facteurs de transcription ciblant les gènes spécifiques du métabolisme des lipides (Fève et al. Grégoire et al. ; 1998). Ce processus est ainsi couramment décrit sous le terme d'adipogenèse ou de différenciation adipocytaire ou encore de conversion adipocytaire. Il a récemment été mis en évidence que les pré-adipocytes étaient présents dans le tissu adipeux tout au long de l'existence de l'être humain, représentant de 15 à 50% des cellules de ce tissu chez l'adulte (Yu et al. 2004 ; Guo et al. 2006). Ainsi, à tout moment de la vie, des pré-adipocytes peuvent se différencier en adipocytes, conduisant alors à un renouvellement des cellules du tissu adipeux ou à son expansion. Il semblerait cependant que certaines périodes du développement humain soient primordiales dans l'augmentation du nombre d'adipocytes et par conséquent dans la constitution de la masse graisseuse. La première année de la vie et la période de l'adolescence sont notamment deux périodes importantes au cours desquelles le tissu adipeux semble plus soumis à des changements importants de sa constitution (Smith et al. 2006).

Le vieillissement cutané, et plus particulièrement le vieillissement facial, qui préoccupe beaucoup les individus, se caractérise par deux phénomènes tissulaires, la ptose et l'atrophie.

- La ptose est liée à une perte de tonicité de tous les éléments de la peau (tissu, ligaments, muscles). Le tissu cutané perd son élasticité et ptose avec la graisse sous-jacente (Dumont et al. 2007). Les caractéristiques visuelles de ce phénomène ne sont autres que la visualisation exacerbée du pli nasogénien, la descente des bajoues,...
- L'atrophie survient dans un second temps et joue un rôle prépondérant dans les modifications volumétriques survenant au niveau du visage avec le vieillissement. Elle concerne certes le tissu adipeux, mais également toutes les autres couches structurelles (derme, épiderme, muscle,...). Ce phénomène d'atrophie du tissu adipeux aggrave en plus la ptose par la création d'un déséquilibre « contenu-contenant » (Dumont et al. 2007). Les conséquences visibles de ce phénomène sont l'observation de visages creux, d'un manque de volume et/ou de contenance de la peau. Le tissu adipeux subit en effet de nombreux changements physiologiques et métaboliques au cours du vieillissement. L'atrophie du tissu adipeux observée s'explique par différents phénomènes qui se mettent en place et qui affectent tant les adipocytes que les pré-adipocytes. Au niveau des adipocytes, une diminution de leur taille est observée, alors que leur nombre reste constant (I. Karagiannides et al. 2001, 2006). Celle-ci peut s'expliquer par l'observation d'une diminution à la fois de leur capacité à accumuler des lipides et à faire la lipolyse. Les pré-adipocytes sont également touchés par le processus de vieillissement. En effet, leur capacité à se différencier en adipocytes et, par conséquent aux adipocytes en résultant à accumuler des lipides est diminuée (I. Karagiannides et al. 2001, 2006). De plus, le tissu adipeux, avec ses fortes concentrations en acides gras libres cytotoxiques, couplées à un grand nombre de macrophages et de cytokines pro-inflammatoires, peut infliger des dommages aux pré-adipocytes quiescents, entraînant une différenciation en adipocyte incomplète (Guo et al. 2006; I. Karagiannides et al. 2006). Les cellules non fonctionnelles ainsi créées ont été décrites par Kirkland (Kirkland et al. 2002) et appelées MAD (Mesenchymal Adipocyte-like Default) cells. Ces MAD cells sont des cellules sont plus petites et moins répondantes à l'insuline que les adipocytes fonctionnels. Elles se caractérisent aussi par une capacité de métabolisation des acides gras réduite et produisent de plus grandes quantités de cytokine TNF$\alpha$. La production de cette cytokine TNF$\alpha$ en large quantité empêche la différentiation des pré-adipocytes adjacents et peut les transformer à leur tour en MAD cells. La perte d'élasticité de la peau humaine, plus particulièrement la perte d'élasticité de certaines parties du corps humain comme les seins, le visage, les joues, les fesses et les paupières, peut s'expliquer par le fait que le derme contient les fibres élastiques et les fibres de collagène, qui déterminent principalement les propriétés viscoélastiques de la peau humaine et que l'hypoderme, couche la plus profonde de la peau humaine agit également sur ces propriétés. En effet, le tissu hypodermique comprime de façon variable le derme en fonction de son épaisseur ; ainsi, une augmentation de l'épaisseur de l'hypoderme peut entrainer une modification des propriétés biomécaniques de la peau et notamment une amélioration de son élasticité. Pour répondre aux attentes des individus désireux d'améliorer l'aspect de leur silhouette, ou de leur buste, ou de leurs mains, ou de leur visage, il existe des solutions de nature chirurgicale. Une de ces techniques est connue sous le nom de « lipo-filling » et consiste à prélever le tissu adipeux dans une partie du corps humain d'un individu pour l'implanter sous la peau d'un autre endroit du corps du même individu que l'on désire corriger par augmentation de volume. Cependant, ces techniques chirurgicales présentent comme inconvénient d'être invasive, coûteuse, et de laisser sur le corps des cicatrices visibles pendant une longue durée. Il existe également de nombreux principes actifs dits "anti-âge" ou "antirides" qui peuvent être administrés de façons diverses à l'être humain. Plusieurs de ces ingrédients « actifs » sont capables de réguler le phénotype et/ou les propriétés de fibroblastes humains normaux. Par exemple, de nombreux principes actifs cosmétiques stimulent la production fibroblastique de collagène présent dans le derme. Ainsi, parmi les dérivés N-acylés d'acides aminés utilisés dans des applications cosmétiques, certains présentent des propriétés biologiques anti-âge, tels que le dipalmitoyl hydroxyproline (SEPILIFT™ DPHP). D'autres principes actifs s'intéressent plus à la protection de l'épiderme, avec des effets anti-radicalaires, ou des effets sur le maintien et/ou l'amélioration des fonctions de l'épiderme selon des mécanismes de différenciation et/ou d'immunité. En revanche, peu de principes actifs anti-âges sont connus pour leur capacité à agir au niveau du tissu adipeux présent dans l'hypoderme en favorisant le métabolisme des cellules le composant, afin de redonner du volume à la peau, et/ou à la « repulper » et/ou à lui redonner de l'élasticité. Parmi ceux-ci, on peut citer le tri-terpenoïde sapogenin phytostérol (décrit dans la demande de brevet WO2008/015639 A2) ou les extraits à base de Commiphora mukul, qui agissent sur des cellules non altérées par le vieillissement. La publication internationale WO90/14429A1 décrit le N-hexadécanoyl isoleucine et enseigne son utilisation pour la préparation de compositions détergentes. La publication JP 2003-096039 décrit un procédé de préparation de sels de N-acyl aminoacides, ainsi que leurs propriétés de surface et leur action fongistatique. Cette publication décrit particulièrement les sels de sodium, de potassium, de triéthylamine et d'arginine du N-hexadécanoyl isoleucine. La publication internationale WO 98/09611 décrit l'utilisation des N-acyl aminoacides comme régulateurs de la physiologie cutanée, et plus particulièrement comme agent stimulant le métabolisme cellulaire et restructurant la barrière cutanée. La publication intitulée « Lipoaminoacides et cosmétologie » de J. Morelle (Parfums cosmétiques savons de France, Paris, vol.3, n° 2, 1 février 1973) décrit l'utilisation des N-acyl aminoacides comme stimulateurs du processus de développement cellulaire pour leurs propriétés antiséborrhéiques, antifongiques et antibactériennes. La publication FR 2 873 575 A1 décrit des compositions cosmétiques comprenant des N-acyl aminoacides utilisées pour procurer un effet antivieillissement de la peau pouvant désigner un effet de restauration de l'élasticité de la peau par amélioration des tissus conjonctifs.

A notre connaissance, aucun N-acyl aminoacide n'a été décrit comme capable de réguler l'activité des cellules présentes dans le tissu adipeux de l'hypoderme, en améliorant la différentiation adipocytaire et/ou en induisant l'augmentation de la surface globale des adipocytes et/ou en restaurant le phénotype adipocytaire. Aucun N-acyl aminoacide n'a non plus été décrit comme capable d'entrainer une augmentation du volume du tissu adipeux de l'hypoderme sur quelconque zone du corps humain, se traduisant alors par une augmentation mesurable du volume de ladite zone, ni comme capable d'entrainer une augmentation de la densité et de l'épaisseur du tissu adipeux de l'hypoderme sur quelconque zone du corps humain, se traduisant alors par une augmentation apparente du volume de ladite zone.

C'est pourquoi, selon un premier aspect, l'invention a pour objet l'utilisation cosmétique de N-hexadécanoyl isoleucine de formule (I) :

$$CH_3\text{-}(CH_2)_{14}\text{-}C(=O)\text{-}NH\text{-}CH(COOH)\text{-}CH(CH_3)\text{-}CH_2\text{-}CH_3 \qquad (I)$$

comme agent « volumateur » et/ou comme agent « repulpant » de la peau humaine.

L'expression « utilisation cosmétique de N-hexadécanoyl isoleucine de formule (I) » désigne notamment une utilisation destinée à embellir et/ou à améliorer l'apparence du corps humain.

Par « N-hexadécanoyl isoleucine de formule (I) comme agent « volumateur » de la peau humaine », on entend tout effet du N-hexadécanoyl isoleucine de formule (I) qui entraine une augmentation du volume du tissu adipeux de l'hypoderme sur quelconque zone du corps humain, se traduisant alors par une augmentation mesurable du volume de ladite zone. L'effet « volumateur » peut être analysé, mesuré et quantifié par échographie ou par la mise en oeuvre de techniques adaptées à la zone du corps humain pour laquelle on souhaite mettre en évidence cet effet. Par exemple, pour mettre en évidence l'effet « volumateur » sur les seins des femmes, on pourra mesurer l'évolution du tour de poitrine.

Par « N-hexadécanoyl isoleucine de formule (I) comme agent « repulpant » de la peau humaine, on entend tout effet du N-hexadécanoyl isoleucine de formule (I) qui entraine une augmentation de la densité et de l'épaisseur du tissu adipeux de l'hypoderme sur quelconque zone du corps humain, se traduisant alors par une augmentation apparente du volume de ladite zone. L'effet « repulpant » ou « redensifiant » du tissu adipeux peut être analysé par la technique de projection de frange ou par échographie.

Dans le cadre de la mise en évidence de l'effet « volumateur » et de l'effet « repulpant » du N-hexadécanoyl isoleucine de formule (I), la demanderesse a mis en évidence l'action du N-hexadécanoyl isoleucine de formule (I) pour réguler l'activité des cellules présentes dans le tissu adipeux de l'hypoderme de la peau humaine. Dans l'expression « pour réguler l'activité des cellules présentes dans le tissu adipeux de l'hypoderme de la peau humaine », on désigne plus particulièrement que le N-hexadécanoyl isoleucine de formule (I) présente l'une, l'autre, plusieurs ou l'ensemble des propriétés suivantes :

- il améliore la différenciation des adipocytes présents dans le tissu adipeux de l'hypoderme de la peau humaine ;
- il augmente la surface globale des adipocytes présents dans le tissu adipeux de l'hypoderme de la peau humaine ;
- il restaure le phénotype adipocytaire dans le tissu adipeux de l'hypoderme de la peau humaine, et par conséquent prévient la génération de cellules altérées dans le tissu adipeux de l'hypoderme de la peau humaine.

Le N-hexadécanoyl isoleucine de formule (I) peut se présenter sous forme acide libre ou sous forme partiellement ou totalement salifiée. Lorsque le N-hexadécanoyl isoleucine est sous forme salifiée, il s'agit notamment de sels alcalins tels que les sels de sodium, de potassium ou de lithium, de sels alcalino-terreux tels que les sels de calcium, de magnésium ou de strontium ; de sel d'ammonium ou de sels d'amino-alcools comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium. De façon générale, le taux de salification du N-hexadécanoyl isoleucine de formule (I) dépendra en autre de son pKA et de la concentration en sels de la composition dans laquelle il est incorporé. Dans l'exposé suivant, par N-hexadécanoyl isoleucine de formule (I), on entend le N-hexadécanoyl isoleucine de formule (I) sous forme libre ou partiellement ou totalement salifiée.

Le N-hexadécanoyl isoleucine de formule (I) est généralement obtenu par N-acylation de l'isoleucine ou de ses sels en utilisant comme agent d'acylation un dérivé activé de l'acide hexadécanoïque de formule :

$$CH_3\text{-}(CH_2)_{14}\text{-}C(=O)\text{-}OH,$$

tel qu'un anhydride symétrique de cet acide, un ester méthylique de cet acide, ou un halogénure d'acide comme le chlorure d'acide ou le bromure d'acide. La réaction d'acylation est connue de l'homme du métier. Elle est décrite par exemple dans la demande internationale publiée sous le numéro WO 98/09611.

Selon un aspect plus particulier, l'invention a pour objet l'utilisation cosmétique de N-hexadécanoyl isoleucine de formule (I) caractérisée en ce que le N-hexadécanoyl isoleucine de formule (I) favorise en outre l'expansion du tissu adipeux de l'hypoderme de la peau humaine.

Par l'expression « favoriser l'expansion du tissu adipeux de l'hypoderme de la peau humaine », on entend désigner toute augmentation de la masse et/ou du volume et/ou de l'épaisseur du tissu adipeux de l'hypoderme de la peau humaine.

Selon un aspect plus particulier, l'invention a pour objet l'utilisation cosmétique de N-hexadécanoyl isoleucine de formule (I), caractérisée en ce que le N-hexadécanoyl isoleucine de formule (I) stimule en outre une augmentation de l'élasticité et/ou un effet « volumateur » et/ou un effet « repulpant » de la peau humaine.

La peau humaine possède des caractéristiques rhéologiques évaluées selon deux paramètres :

- un paramètre élastique,
- un paramètre de tension qui varie selon les différentes zones du corps humain.

Dans le cadre de la présente invention, on entend par « N-hexadécanoyl isoleucine de formule (I) stimule une augmentation de l'élasticité» de la peau humaine tout effet du N-hexadécanoyl isoleucine de formule (I) qui entraine une diminution du paramètre élastique en ballistométrie de la peau humaine, correspondant ainsi à une augmentation de l'élasticité de la peau humaine.

Les propriétés biomécaniques peuvent être analysées, mesurées et quantifiées par des techniques reposant sur l'étude de l'évolution de ces paramètres comme par exemple les techniques faisant intervenir l'utilisation d'un « Cutometer™ » ou d'un « Dermal Torque Meter™ » ou d'un « Ballistomètre™ ».

Selon un second aspect, l'invention a pour objet un procédé de traitement cosmétique du corps humain pour obtenir un effet « volumateur » et/ou « repulpant » de la peau d'une partie du corps humain choisie parmi les seins, le visage, les joues, les fesses et les paupières caractérisé en ce qu'il consiste à appliquer sur ladite partie une composition cosmétique comprenant une quantité efficace de N-hexadécanoyl isoleucine de formule (I).

Par quantité efficace de N-hexadécanoyl isoleucine de formule (I) à appliquer sur la peau d'une partie du corps humain choisie parmi les seins, le visage, les joues, les fesses et le paupières pour obtenir un effet « volumateur » et/ou « repulpant » de la peau de ladite partie, on entend la quantité comprise entre 0,000001% et 0,5% massique, plus particulièrement entre 0,00001% et 0,05% massique, et encore plus particulièrement entre 0,0001% et 0,005% massique de N-hexadécanoyl isoleucine de formule (I).

Selon un aspect plus particulier, l'invention a pour objet un procédé de traitement cosmétique du corps humain tel que défini précédemment caractérisé en ce qu'il permet en outre de favoriser l'expansion du tissu adipeux de l'hypoderme de la peau humaine.

Selon un aspect encore plus particulier, l'invention a pour objet un procédé de traitement cosmétique du corps humain tel que défini précédemment caractérisé en ce que l'application de la quantité efficace de N-hexadécanoyl isoleucine de formule (I) est réalisée sur les seins et/ou sur le visage et/ou sur les joues et/ou sur les fesses et/ou sur les paupières pour obtenir une augmentation de l'élasticité de la peau et/ou un effet volumateur et/ou un effet repulpant.

Dans le procédé de traitement cosmétique du corps humain objet de la présente invention, le N-hexadécanoyl isoleucine de formule (I) est généralement compris dans une composition comprenant des ingrédients cosmétiquement acceptables.

L'expression « cosmétiquement acceptable » utilisée dans la définition des ingrédients associés au N-hexadécanoyl isoleucine de formule (I) dans les compositions mises en oeuvre dans le procédé de traitement cosmétique objet de la présente invention, signifie selon la directive du Conseil de la Communauté Economique Européenne N°76/768/CEE du 27 juillet 1976 modifiée par la directive N°93/35/CEE du 14 juin 1993, que ladite composition comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Les compositions comprenant le N-hexadécanoyl isoleucine de formule (I) mises en oeuvre dans le procédé de traitement cosmétique du corps humain objet de la présente invention, se présentent généralement sous forme de solutions aqueuses ou hydro-alcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

Les compositions comprenant le N-hexadécanoyl isoleucine de formule (I) mises en oeuvre dans le procédé de traitement cosmétique du corps humain objet de la présente invention, sont administrées plus particulièrement par voie topique directe ou indirecte, par l'intermédiaire d'un support textile ou de matériaux non tissés tels que décrits précédemment.

Dans les compositions comprenant le N-hexadécanoyl isoleucine de formule (I) mises en oeuvre dans le procédé de traitement cosmétique du corps humain objet de la présente invention telles que définies ci-dessus, le N-hexadécanoyl isoleucine de formule (I) est généralement mis en oeuvre en une quantité comprise entre 0,01% et 10% massique pour 100% de la masse de ladite composition, plus particulièrement entre 0,1% à 5% massique, et tout particulièrement entre 1% et 5% massique.

De façon générale, le N-hexadécanoyl isoleucine de formule (I) est associé à de nombreux types d'adjuvants ou de principes actifs utilisés dans les compositions cosmétiques mises en oeuvre dans le procédé de traitement cosmétique du corps humain objet de la présente invention, qu'il s'agisse, de corps gras, de solvants organiques, d'épaississants, de gélifiants, d'adoucissants, d'agents tensioactifs détergents, d'agents surgraissants, de tensioactifs épaississants et/ou gélifiants, d'antioxydants, d'opacifiants, de stabilisants, de moussants, de parfums, de tensioactifs émulsionnants, d'agents hydrotropes, d'agents plastifiants, d'agents surgraissants, d'agents de texture, de pigments, de séquestrants, de chélateurs, de conservateurs, de filtres chimiques ou de filtres minéraux, d'huiles essentielles, de matières colorantes, de pigments, d'actifs hydrophiles ou lipophiles, d'humectants, par exemple la glycérine, de conservateurs, de colorants, de parfums, d'actifs cosmétiques, de filtres solaires minéraux ou organiques, de charges minérales comme les oxydes de fer, oxydes de titane et le talc, de charges synthétiques comme les nylons et les poly(méthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer au N-hexadécanoyl isoleucine de formule (I) dans les compositions cosmétiques mises en oeuvre dans le procédé de traitement cosmétique du corps humain objet de la présente invention, on peut citer, les huiles minérales telles que l'huile de paraffine, l'huile de vaseline, les isoparaffines ou les huiles blanches minérales, les huiles d'origine animale, telles que le squalène ou le squalane, les huiles végétales, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse que l'on peut associer au N-hexadécanoyl isoleucine de formule (I) dans les compositions cosmétiques mises en oeuvre dans le procédé de traitement cosmétique du corps humain objet de la présente invention, on peut citer les alcools gras ou les acides gras.

Comme exemple de cires que l'on peut associer au N-hexadécanoyl isoleucine de formule (I) dans les compositions cosmétiques mises en oeuvre dans le procédé de traitement cosmétique du corps humain objet de la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Comme exemple de polymères épaississants et/ou émulsionnant que l'on peut associer au N-hexadécanoyl isoleucine de formule (I) dans les compositions cosmétiques mises en oeuvre dans le procédé de traitement cosmétique du corps humain objet de la présente invention, on peut citer par exemple les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes.

Parmi les polymères de type polyélectrolytes que l'on peut associer au N-hexadécanoyl isoleucine de formule (I), il y a par exemple, les copolymères de l'acide acrylique et de l'acide 2-méthyl [(1-oxo 2-propènyl) amino] 1-propanesulfonique (AMPS), les copolymères de l'acrylamide et de l'acide 2-méthyl [(1-oxo 2-propényl) amino] 1-propanesulfonique, les copolymères de l'acide 2-méthyl [(1-oxo 2-propényl) amino] 1-propanesulfonique et de l'acrylate de (2-hydroxy éthyle), l'homopolymère de l'acide 2-méthyl [(1-oxo 2-propényl) amino] 1-propanesulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800 et SIMULGEL™ A par la demanderesse.

Comme exemples d'émulsionnants que l'on peut associer au N-hexadécanoyl isoleucine de formule (I) dans les compositions cosmétiques mises en oeuvre dans le procédé de traitement cosmétique du corps humain objet de la présente invention, on peut citer par exemple les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435 et 2 804 432.

Comme exemples de principe actif que l'on peut associer au N-hexadécanoyl isoleucine de formule (I) dans les compositions cosmétiques mises en oeuvre dans le procédé de traitement cosmétique du corps humain objet de la présente invention, on peut citer les composés ayant une action éclaircissante ou dépigmentante tels que par exemple l'arbutine, l'acide kojique, l'hydroquinone, l'acide ellagique, la vitamine C, le magnésium ascorbyl phosphate, les extraits de polyphénols, les dérivés de polyphénols glycosylés comme le Rosmarinyl glucoside, les extraits de raisin, les extraits de pin, les extraits de vin, les extraits d'olives, les extraits de mare, les protéines N-acylées, les peptides N-acylés, les acides aminés N-acylés, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, la vitamine E et ses dérivés, les minéraux, les enzymes, les co-enzymes, comme, le Coenzyme Q10, les hormones ou "hormone like", les extraits de soja par exemple, la Raffermine™, les extraits de blé par exemple la Tensine™ ou la Gliadine™, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, comme l'extrait de quinoa commercialisé sous l'appellation ADIPOLESS™, comme l'extrait de pruche canadienne commercialisé sous l'appellation SERENIKS™ 207, comme la composition comprenant du Lauroyl Proline commercialisée sous l'appellation ADIPOSLIM™, les actifs ayant une activité antimicrobienne ou une action purifiante vis à vis des peaux grasses tels que le LIPACIDE™ PVB, les actifs ayant une propriété énergisante ou stimulante comme le SEPITONIC™ M3 ou le Physiogényl™ le panthénol et ses dérivés comme le SEPICAP™ MP, les actifs anti-âge comme le SEPILIFT™ DPHP, le LIPACIDE™ PVB, le SEPIVINOL™, le SEPIVITAL™, les actifs hydratants comme le SEPICALM™ S, le SEPICALM™ VG et le SEPILIFT™ DPHP, les actifs anti-âge " anti-photo vieillissement ", les actifs protecteurs de l'intégrité de la jonction dermo-épidermique, les actifs augmentant la synthèse des composants de la matrice extracellulaire, les actifs ayant une activité amincissante, raffermissante ou drainante comme la caféine, la théophylline, l'AMPc, le thé vert, la sauge, le ginko biloba, le lierre, le marron d'inde, le bambou, le ruscus, le petit houx, la centella asiatica, la bruyère, l'ulmaine, le fucus, le romarin, la saule, des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

Comme exemples d'agents de texture que l'on peut associer au N-hexadécanoyl isoleucine de formule (I) dans les compositions cosmétiques mises en oeuvre dans le procédé de traitement cosmétique du corps humain objet de la présente invention, on peut citer des dérivés N-acylés d'acides aminés, comme par exemple la lauroyl lysine commercialisée sous l'appellation AMINOHOPE™ LL par la société AJINOMOTO, l'octenyl starch succinate commercialisé sous l'appellation DRYFLO™ par la société NATIONAL STARCH, le myristyl polyglucoside commercialisé par SEPPIC sous l'appellation MONTANOV™ 14, les fibres de cellulose, les fibres de coton, les fibres de chitosane, le talc, la séricite, le mica.

Comme exemples d'agents opacifiants et/ou nacrants que l'on peut associer au N-hexadécanoyl isoleucine de formule (I) dans les compositions cosmétiques mises en oeuvre dans le procédé de traitement cosmétique du corps humain objet de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras.

Comme exemples de tensioactifs épaississants et/ou gélifiants que l'on peut associer au N-hexadécanoyl isoleucine de formule (I) dans les compositions cosmétiques mises en oeuvre dans le procédé de traitement cosmétique du corps humain objet de la présente invention, on peut citer :

- Les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATET™ DOE120.
- Les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.
- Les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples de filtres solaires que l'on peut associer au N-hexadécanoyl isoleucine de formule (I) dans les compositions cosmétiques mises en oeuvre dans le procédé de traitement cosmétique du corps humain objet de la présente invention, on peut citer tous ceux figurant dans la directive cosmétique 76/768/CEE modifiée annexe VII.

Les études expérimentales suivantes illustrent l'invention sans toutefois la limiter.

## 1 - Etudes in vitro

Les études in-vitro mettent en évidence que le N-hexadécanoyl isoleucine de formule (I) régule l'activité des cellules du tissu adipeux de l'hypoderme en permettant une stimulation de la différenciation de pré-adipocytes humains normaux dans un modèle reproduisant des conditions d'insuffisance de stimulation adipogénique, en permettant la stimulation de l'augmentation de la surface adipocytaire, et en permettant de restaurer le phénotype adipocytaire de pré-adipocytes humains normaux « jeunes » dans un modèle reproduisant des conditions de vieillissement.

### 1.1 - Mise en évidence de l'efficacité stimulatrice du N-hexadécanoyl isoleucine sur la différenciation de pré-adipocytes humains normaux dans un modèle in vitro reproduisant des conditions d'insuffisance de stimulation adipogénique, et sur l'efficacité stimulatrice du N-hexadécanoyl isoleucine sur l'augmentation de la surface adipocytaire.

Protocole

Des pré-adipocytes humains normaux issus de déchets opératoires abdominaux d'un donneur de sexe féminin, âgé de 38 ans, ont été cultivés en monocouche jusqu'à confluence en présence d'un milieu de culture comprenant du PBM-2 (Pre-adipocyte Basal Medium-2) fourni par la société Lonza, une quantité de 10% volumique de FBS (Foetal Bovine Serum) pour 100% du volume du milieu de culture, de la L-glutamine et de la gentamicine.

Leur différenciation en adipocytes a été induite par addition d'un milieu de différenciation «appauvri», comprenant un mélange d'insuline, de déxamethasone, d'IBMX (Isobutyl méthyl xanthine) et d'indométhacine, et utilisé à un taux de dilution de 1/10ème par rapport à un milieu de différenciation «standard». Cette phase de différenciation par addition d'un milieu de différenciation «appauvri» est réalisée en présence des produits testés dans le cadre de la présente démonstration, à savoir le N-hexadécanoyl isoleucine, l'acide hexadécanoïque, l'isoleucine, un mélange d'acide hexadécanoïque et d'isoleucine dans un rapport massique acide hexadécanoïque/isoleucine de 65/35, et également en absence de produits de façon à obtenir un «témoin différencié en milieu appauvri».

Une phase de différenciation est également réalisée sur des pré-adipocytes humains tels que décrits précédemment et cultivés en monocouches tel que décrits précédemment, mais en présence d'un milieu de différenciation « standard », à savoir un mélange non dilué d'insuline, de déxamethasone, d'IBMX (IsoButylMéthylXanthine) et d'indométhacine, de façon à obtenir un « témoin différencié en milieu standard ».

Une fois la maturité des pré-adipocytes atteinte, à savoir après 15 jours suivant l'induction précédemment décrite, les cultures ont été rincées à l'aide d'un tampon PBS (Phosphate Buffered Saline) et un marquage des vésicules lipidiques intra-cytoplasmiques a été réalisé à l'aide d'une sonde fluorescente viable spécifique, le BODIPY ($500\mu$g/mL). Les noyaux des pré-adipocytes différenciés ont été également marqués à l'aide d'un réactif de Hoechst (bisbenzimide), également fluorescent à une concentration de $2\mu$g/mL, commercialisé par la société Sigma.

Trois photographies par puits ont été réalisées à l'aide d'un microscope inversé à épifluorescence de marque Zeiss Axiovert™ 25, muni d'un objectif x20.

Résultats

Pour chaque essai de produits testés, témoins inclus, les images obtenues avec le microscope inversé à épifluorescence sont analysées à l'aide du logiciel LUCIA, permettant la quantification du marqueur fluorescent Bodipy, afin de réaliser :

-   une mesure de la surface des gouttelettes lipidiques (Sg),
-   une mesure du nombre de cellules différenciées (Nc) et,
-   une mesure du nombre de noyaux cellulaires totaux (Nnc).

Pour chaque essai, on calcule alors le niveau de différenciation adipocytaire (D) en rapportant la surface des gouttelettes lipidiques au nombre de noyaux totaux selon la formule suivante : (D) = (surface des gouttelettes lipidiques) / (nombre de noyaux cellulaires totaux)

On calculera ainsi :

-   le niveau de différenciation adipocytaire du témoin différencié en milieu appauvri, (noté par la suite Dma) ;
-   le niveau de différenciation adipocytaire du témoin différencié en milieu standard ou complet, (noté par la suite Dmc) ;
-   le niveau de différenciation adipocytaire des produits testés (N-hexadécanoyl isoleucine, acide hexadécanoïque, isoleucine, mélange acide hexadécanoïque/ isoleucine dans un rapport massique de 65/35) (noté par la suite Dpt).

Pour chaque produit testé, on calcule ensuite :

a) le pourcentage de restauration de la différenciation adipocytaire (noté par la suite RDA) selon la formule suivante :

$$RDA = [(Dpt\text{-}Dma) / (Dmc\text{--}Dma)] \times 100$$

b) la surface adipocytaire (SA) selon la formule suivante :

$$(SA) = [(\text{surface des gouttelettes lipidiques Sg}) / (\text{nombre de cellules différenciées Nc})] \times 100$$

c) le pourcentage d'augmentation de la surface adipocytaire (noté par la suite ASA) par rapport au témoin traité avec milieu appauvri selon la formule :

$$ASA = [\text{Surface adipocytaire du produit (SApt)} / \text{Surface adipocytaire du témoin traité avec le milieu appauvri (SAma)}] \times 100.$$

On réalise ensuite une analyse statistique sur les données (D) et (SA) à l'aide d'un test de Student bilatéral à variance inégale. Ainsi la population de chacune des conditions expérimentales testées pour les paramètres (D) et (SA) est comparée à la population de la condition de différenciation en présence du milieu appauvri de différenciation. Un seuil d'erreur (p) est fixé à 5%. Si p ≤ 0,05, les deux populations étudiées sont considérées comme significativement différentes, et la condition expérimentale étudiée présente alors un effet par rapport à la condition de différenciation en milieu appauvri. Si p > 0,05, les deux populations sont considérées comme ne présentant pas de différences et par conséquent, la condition expérimentale étudiée ne présente pas un effet par rapport à la condition de différenciation en milieu appauvri.

Les résultats obtenus sont consignés dans le tableau 1 ci-dessous :

Tableau 1

| Produits testés | RDA | ASA |
|---|---|---|
| Témoin différencié en milieu appauvri | 0% | 100% |
| Témoin différencié milieu standard | 100%* | 194%* |
| N-hexadécanoyl isoleucine (5 μg/ml en extrait sec) | 63%* | 215%* |
| N-hexadécanoyl isoleucine (10 μg/ml en extrait sec) | 85%* | 214%* |
| Acide hexadécanoïque (3,3 μg/ml) | <10% | 41%* |
| Acide hexadécanoïque (6,5 μg/ml) | <10% | 62% |
| Isoleucine (1,7 μg/ml en extrait sec) | <10% | 57% |
| Isoleucine (3,3 μg/ml en extrait sec) | <10% | 117% |
| Acide hexadécanoïque /Isoleucine (65/35) (5μg/ml en extrait sec) | <10% | 88% |
| Acide hexadécanoïque /Isoleucine (65/35) (10μg/ml en extrait sec) | <10% | 49% |
| (*) : Résultat statistiquement significatif par rapport au témoin différencié en milieu appauvri (test de Student bilatéral à variance inégale) avec p≤ 0,05 | | |

Les essais réalisés selon le protocole décrit précédemment et compris dans le tableau 1 montrent que le N-hexadécanoyl isoleucine permet une RDA de 63% pour une dose de 5μg/ml en extrait sec de N-hexadécanoyl isoleucine, et une RDA de 85% pour une dose de 10μg/ml en extrait sec.

Lorsque l'acide hexadécanoïque est mis en oeuvre à des doses de 3,3 μg/ml en extrait sec et de 6,5 μg/ml en extrait sec, et lorsque l'isoleucine est mise en oeuvre à des doses de 1,7 μg/ml en extrait sec et de 3,3 μg/ml en extrait sec, la RDA est inférieure à 10% et considérée comme non significative. Il en est de même, pour les essais avec un mélange d'acide hexadécanoïque et d'isoleucine dans une proportion massique acide hexadécanoïque / isoleucine de 65/35, à des doses de 5 μg/ml en extrait sec et de 10 μg/ml en extrait sec.

Ces résultats montrent donc que la structure N-acylée du N-hexadécanoyl isoleucine permet une RDA efficace.

Les essais montrent également que le N-hexadécanoyl isoleucine permet d'obtenir un taux d'augmentation de la surface adipocytaire de 215% pour une dose de 5 μg/ml en extrait sec et de 214% pour une dose de 10 μg/ml en extrait sec, contre 194% pour le témoin différencié en milieu standard. D'autre part, lorsque l'acide hexadécanoïque est mis en oeuvre à des doses de 3,3 μg/ml en extrait sec et de 6,5 μg/ml en extrait sec, et lorsque l'isoleucine est mise en oeuvre à des doses de 1,7 μg/ml en extrait sec et de 3,3 μg/ml en extrait sec dans des essais réalisés selon protocole décrit précédemment, les taux d'ASA obtenus sont nettement inférieurs à celui obtenu pour le témoin différencié en milieu standard, et dans la plupart des cas, ils ont aussi inférieurs au taux d'ASA obtenu pour le témoin différencié en milieu appauvri. Le même constat est observé pour les essais réalisés avec un mélange d'acide hexadécanoïque et d'isoleucine dans une proportion massique acide hexadécanoïque/isoleucine de 65/35, à des doses de 5 μg/ml en extrait sec et de 10 μg/ml en extrait sec.

Ces résultats montrent donc que la structure N-acylée du N-hexadécanoyl isoleucine permet une ASA efficace.

**1.2 - Mise en évidence de l'effet restaurateur d'un phénotype adipocytaire par le N-hexadécanoyl isoleucine à partir de pré-adipocytes humains normaux dans un modèle in vitro reproduisant des conditions de vieillissement.**

Le modèle in vitro utilisé consiste en un vieillissement accéléré de pré-adipocytes humains normaux par «passages» successifs afin de générer des cellules présentant un phénotype et les caractéristiques de MAD cells après différenciation. Par «passage», ou «repiquage» ou «amplification», on désigne ici l'opération connue de l'homme du métier consistant à amplifier les cellules concernées de façon successive par des cycles de culture. Ainsi, par pré-adipocytes de «passage R0», on désigne les pré-adipocytes qui n'ont subi aucune opération de passage telle que définie précédemment. Par pré-adipocytes de «passage R1», on désigne les pré-adipocytes qui ont subi une seule opération de passage telle que définie précédemment. Par pré-adipocyte de «passage R7», on désigne les pré-adipocytes qui ont subi 7 opérations successives de passage dans des conditions opératoires identiques. De ce fait, on considérera les pré-adipocytes de passage R1 comme représentatifs de pré-adipocytes d'un sujet «jeune» car ils ne présentent pas d'altérations morphologiques et phénotypiques dues aux étapes successives de passage. D'autre part, on considérera les pré-adipocytes de passage R7 comme représentatifs d'un sujet artificiellement vieilli car ils présentent des altérations morphologiques et phénotypiques dues aux étapes successives de passage. 3 protocoles différents ont été mis en oeuvre pour mettre en évidence l'efficacité du N-hexadécanoyl isoleucine à restaurer le phénotype de pré-adipocytes différenciés et artificiellement vieillis.

**Protocole 1.**

**a) -** Préparation du témoin «jeune».

i) - Des pré-adipocytes humain de passage R1 ont été cultivés en monocouche pendant 5 jours jusqu'à confluence. Leur différenciation en adipocytes a été induite par l'addition d'un mélange standard de différenciation comprenant un mélange d'insuline, de déxamethasone, d'IBMX (Isobutyl méthyl Xanthine) et d'indométhacine.

ii) - Après une durée de 15 jours suivant l'étape d'induction décrite ci-dessus, les cultures préparées sont rincées à l'aide d'un tampon PBS (Phosphate Buffered Saline) puis fixées à l'aide d'une solution comprenant, pour 100% de la masse de ladite solution 4% massique de formaldéhyde et 0,1% massique de Triton X-100, détergent de synthèse couramment utilisé en biologie.

iii) - Un marquage des vésicules lipidiques intra-cytoplasmiques des cultures cellulaires précédemment préparées est ensuite réalisé à l'aide d'une quantité de 0,2% massique du marqueur Oil-Red-O commercialisé par la société Sigma.

iv) - Les tapis cellulaires contenant les vésicules lipidiques des cultures cellulaires précédemment décrites et marquées comme indiquée ci-dessus sont ensuite lysés avec du diméthyl sulfoxyde (ou DMSO).

v) - La quantité de marquage des vésicules lipidiques des cultures cellulaires marquée et lysées comme préparées à l'issue de l'étape iv) du présent protocole est ensuite estimé par la lecture de la densité optique (DO) de ces lysats à l'aide d'un spectrophotomètre à une longueur d'onde de 540nm.

La densité optique ainsi mesurée pour le témoin jeune est notée « $DO_{R1}$ ».

**b) -** Préparation du témoin « vieilli ».

Les étapes du protocole i) à v) décrites dans le paragraphe a) sont appliquées à des pré-adipocytes humains de passage R7, de façon à obtenir une densité optique pour le témoin « vieilli » noté « $DO_{R7}$ ».

**c) -** Evaluation de l'effet du N-hexadécanoyl isoleucine sur des pré-adipocytes humains de passage R7.

Des pré-adipocytes humain de passage R7 ont été cultivés en monocouches pendant 5 jours jusqu'à confluence. Leur différenciation en adipocytes est induite par l'addition d'un mélange standard de différenciation comprenant un mélange d'insuline, de déxamethasone, d'IBMX (Isobutyl méthyl Xanthine) et d'indométhacine, en présence de N-hexadécanoyl isoleucine. Les étapes ii) à v) telles que décrites au paragraphe a) sont ensuite mises en oeuvre, de façon à obtenir une densité optique pour le N-hexadécanoyl isoleucine notée « $DO_{HIL}$ ».

**d) -** Calcul du taux de restauration phénotypaire

La mesure des différentes densités optiques permet de calculer un taux de restauration phénotypaire (noté ci-après TRP) pour le N-hexadécanoyl isoleucine selon la formule suivante :

$$\text{TRP (\%)} = [(DO_{HIL} - DO_{R7}) / (DO_{R1} - DO_{R7})] \times 100$$

On réalise ensuite une analyse statistique sur les données de densité optique à l'aide d'un test de Student bilatéral à variance inégale. Ainsi, la population de chacune des conditions expérimentales testées est comparée à la population de la condition de différenciation des pré-adipocytes R7. Un seuil d'erreur (p) est fixé à 5%. Si p est inférieur ou égal à 0,05, les deux populations étudiées sont considérées comme significativement différentes, et par conséquent la condition expérimentale étudiée est considérée comme présentant un effet par rapport à la condition de différenciation des pré-adipocytes R7. Si p est strictement supérieur à 0,05, les deux populations sont considérées comme ne présentant pas de différence et par conséquent la condition expérimentale étudiée est considérée comme ne présentant pas d'effet par rapport à la condition de différenciation des pré-adipocytes R7.

Les TRP obtenus pour différentes concentrations en N-hexadécanoyl sont consignés dans le tableau 2 ci-dessous.

Tableau 2

| Produits testés | TRP |
|---|---|
| Témoin R7 différencié | 0% |
| Témoin R1 différencié | 100%* |
| N-hexadécanoyl isoleucine (5 $\mu$g/ml en extrait sec) | 13%* |
| N-hexadécanoyl isoleucine (10 $\mu$g/ml en extrait sec) | 16%* |
| (*) : Résultats statistiquement significatifs par rapport au témoin différencié en milieu appauvri (test de Student bilatéral à variance inégale) avec p$\leq$ 0,05 | |

## Protocole 2.

Les étapes des paragraphes a) et b) du protocole 1 sont mises en oeuvres à partir de pré-adipocytes humains de passage R1 et de pré-adipocytes humains de passage R7 de façon à obtenir un témoin « jeune » et un témoin « vieilli » et les densités optiques $DO_{R1}$ et $DO_{R7}$ associées. L'évaluation de l'effet du N-hexadécanoyl isoleucine sur des pré-adipocytes humains de passage R7 est réalisée selon une variante du mode opératoire du protocole 1, dont la première étape consiste en une culture des pré-adipocytes humains de passage R7 en monocouche pendant 5 jours jusqu'à confluence en présence de N-hexadécanoyl isoleucine. Leur différenciation en adipocytes est induite par l'addition d'un mélange standard de différenciation comprenant un mélange d'insuline, de déxamethasone, d'IBMX (Isobutyl méthyl Xanthine) et d'indométhacine. Les étapes ii) à v) telles que décrites au paragraphe a) du protocole 1 sont ensuite mises en oeuvre, de façon à obtenir une densité optique pour le N-hexadécanoyl isoleucine notée « $DO_{HIL}$ ».

La mesure des différentes densités optiques permet de calculer un taux de restauration phénotypaire (TRP) pour le N-hexadécanoyl isoleucine à la suite de la mise en oeuvre du Protocole 2 selon la formule suivante :

$$TRP\ (\%) = [(DO_{HIL} - DO_{R7}) / (DO_{R1} - DO_{R7})]\ x\ 100$$

Les TRP obtenus pour différentes concentrations en N-hexadécanoyl isoleucine selon le Protocole 2 sont consignés dans le tableau 3 ci-dessous.

Tableau 3

| Produits testés | TRP |
|---|---|
| Témoin R7 différencié | 0% |
| Témoin R1 différencié | 100%* |
| N-hexadécanoyl isoleucine (5 $\mu$g/ml en extrait sec) | 27%* |
| N-hexadécanoyl isoleucine (10 ($\mu$g/ml en extrait sec) | 17%* |
| (*) : Résultats statistiquement significatifs par rapport au témoin différencié en milieu appauvri (test de Student bilatéral à variance inégale) avec p$\leq$ 0,05 | |

**Protocole 3**

Les étapes des paragraphes a) et b) du protocole 1 sont mises en oeuvre à partir de pré-adipocytes humains de passage R1 et de pré-adipocytes humains de passage R7 de façon à obtenir un témoin « jeune » et un témoin « vieilli » et les densités optiques $DO_{R1}$ et $DO_{R7}$ associées.

L'évaluation de l'effet du N-hexadécanoyl isoleucine sur des pré-adipocytes humains de passage R7 est réalisée selon une variante du mode opératoire du protocole 2, dont la première étape consiste en une culture des pré-adipocytes humains de passage R7 en monocouche pendant 5 jours jusqu'à confluence en présence de N-hexadécanoyl isoleucine.

Leur différenciation en adipocytes est induite par l'addition d'un mélange standard de différenciation comprenant un mélange d'insuline, de déxamethasone, d'IBMX (Isobutyl méthyl Xanthine) et d'indométhacine, en présence de N-hexadécanoyl isoleucine. Les étapes ii) à v) telles que décrites au paragraphe a) du protocole 1 sont ensuite mises en oeuvre, de façon à obtenir une densité optique pour le N-hexadécanoyl isoleucine notée « $DO_{HIL}$ ».

La mesure des différentes densités optiques permet de calculer un TRP pour le N-hexadécanoyl isoleucine à la suite de la mise en oeuvre du Protocole 3 selon la formule suivante :

$$\text{TRP (\%)} = [(DO_{HIL} - DO_{R7})/ (DO_{R1} - DO_{R7})] \times 100$$

Les TRP obtenus pour différentes concentrations en N-hexadécanoyl isoleucine selon le Protocole 2 sont consignés dans le tableau 4 ci-dessous.

Tableau 4

| Produits testés | TRP |
|---|---|
| Témoin R7 différencié | 0% |
| Témoin R1 différencié | 100%* |
| N-hexadécanoyl isoleucine (5 $\mu$g/ml en extrait sec) | 36%* |
| N-hexadécanoyl isoleucine (10 $\mu$g/ml en extrait sec) | 30%* |
| (*) : Résultats statistiquement significatifs par rapport au témoin différencié en milieu appauvri (test de Student bilatéral à variance inégale) avec p$\leq$ 0,05 | |

**Conclusion**

Quel que soit le protocole mis en oeuvre, les essais expérimentaux ont montré un effet bénéfique du N-hexadécanoyl isoleucine sur la restauration d'un phénotype adipocytaire en condition de vieillissement artificiel. Ainsi, à une dose de 5 $\mu$g/ml en extrait sec de N-hexadécanoyl isoleucine, cet effet a été estimé par un taux de restauration phénotypaire de 13% selon le Protocole 1, par un taux de restauration phénotypaire de 27% selon le Protocole 2, et par un taux de restauration phénotypaire de 36% selon le Protocole 3.

**2 - Etudes in vivo**

Les études expérimentales suivantes illustrent l'invention sans toutefois la limiter.

Les études in-vivo mettent en évidence que l'utilisation cosmétique du N-hexadécanoyl isoleucine de formule (I) et la mise en oeuvre d'un procédé de traitement cosmétique du corps humain permettent de générer des effets « volumateurs » et/ou « repulpants » de la peau humaine et/ou permettant d'augmenter l'élasticité de la peau.

**2.1 - Mise en évidence de l'effet « volumateur » et de l'effet « repulpant » d'une composition cosmétique comprenant du N-hexadécanoyl isoleucine sur les seins.**

**a) - Préparation d'une émulsion huile-dans-eau (H/E) comprenant du N-hexadécanoyl isoleucine (E1) et d'une émulsion huile-dans-eau « placébo » (E2).**

Les émulsions (E1) et (E2) sont préparées de la manière suivantes :

Etape 1) : Les composants de la phase huileuse et le système émulsionnant sont introduits successivement dans un bêcher, à une température de 80°C et sont soumis à une agitation mécanique pendant une durée de 15 minutes, à l'aide d'un agitateur muni d'un mobile de type « ancre », à une vitesse de 80 tours/minute à une température de 80°C, de façon à former un mélange homogène.

Etape 2) : Le polymère épaississant est ajouté sur le mélange obtenu à l'issue de l'étape 1) à une température de 80°C ainsi que la phase aqueuse, préalablement préparée à température ambiante par mélange de ses composants. Le mélange résultant est ensuite soumis à une agitation par l'intermédiaire d'un émulseur de type rotor-stator, commercialisé par la société SILVERSON, pendant une durée de 4 minutes à une vitesse de 3000 tours/minute à une température de 80°C.

Etape 3) : Le mélange obtenu à l'issue de l'étape 2) est refroidi pendant 10 minute à une température de 30°C, et maintenu sous agitation pendant la durée de refroidissement par l'intermédiaire d'un agitateur mécanique muni d'un mobile de type « ancre », à une vitesse de 80 tours/minute.

Etape 4) : l'agent conservateur Neolone MxP et le parfum « plaisir », sont ensuite ajoutés sur le mélange issu de l'étape 3) à une température de 30°C. Le pH est ensuite ajusté une valeur de 6,5 par ajout de triéthanolamine à 50%.

Les émulsions huile-dans-eau (E1) et (E2) sont de compositions (massiques) suivantes :

Tableau 5

| | (E1) | (E2) |
|---|---|---|
| **Phase huileuse:** | | |
| Paraffine liquide | 8,00% | 8,00% |
| Beurre de Karité | 1,50% | 1,50% |
| DUB DONPG(1) | 5,00% | 5,00% |
| N-hexadécanoyl isoleucine | 1,00% | 0% |
| **Polymère épaisssissant** | | |
| SIMULGEL™ INS 100(2) | 1,00% | 1,00% |
| **Phase aqueuse** | | |
| - Eau | Qs 100% | Qs 100% |
| - Triéthanolamine à 50% | Qs pH = 6,5 | Qs pH = 6,5 |
| - Glycérine | 3,00% | 3,00% |
| **Système émulsionnant** | | |
| MONTANOV™ 202(3) | 2,00% | 2,00% |
| **Additifs** | | |
| - Neolone™ MxP(4) | 0,50% | 0,50% |
| - Parfum Plaisir | 0,10% | 0,10% |
| (1) : Néopentyl Glycol Diéthyl hexanoate commercialisé par la société Stéarinerie Dubois. (2) : Latex inverse épaississant (Dénomination INCI : hydroxyethylacrylate/Sodium acryloyldimethyl taurate copolymer et isohexadecane et Polysorbate 60) commercialisé par la société SEPPIC. (3) : Composition auto-émulsionnable telle que celles décrites dans EP 0 977 626, à base d'alcool arachydilique, d'alcool béhénique et d'arachidyl glucosides, commercialisée par la société SEPPIC. (4) : Mélange conservateur comprenant du phenoxyéthanol, du méthyl paraben, du propyl paraben et du méthyl isothiazolinone, commercialisé par la société Röhm & Haas. | | |

**b) - Evaluation de l'effet « volumateur » et de l'effet « repulpant » des émulsions huile-dans-eau (E1) et (E2) sur les seins**

Ces évaluations sont réalisées en mesurant les critères suivants :

- Mesure de l'évolution centimétrique du tour de poitrine (Mtp),
- Mesure de l'évolution de l'épaisseur de l'hypoderme du sein par échographie de profondeur ;
- Mesure de l'évolution du volume des seins à l'aide de la technique de projection de franges (Primos Body™).

Ces évaluations ont été menées sur une population de 44 volontaires de sexe féminin, d'âge moyen de 26 ans, ayant une poitrine de bonnet A (à savoir une différence de 2,5 cm entre la valeur du tour de poitrine mesurée au niveau des mamelons des deux seins et la valeur du tour de poitrine mesurée à la base des deux seins) qui a été divisée en 2 groupes égaux de 22 volontaires (groupe I et groupe II). Chaque membre du groupe (I) a appliqué sur ses 2 seins de façon biquotidienne et pendant une durée de 28 jours l'émulsion (E1) et chaque membre du groupe (II) a appliqué sur ses 2 seins de façon biquotidienne et pendant une durée de 28 jours l'émulsion (E2).

**b1) -** Evaluation de l'effet « volumateur » du N-hexadécanoyl isoleucine par mesure de l'évolution du tour de poitrine

Protocole

Avant le début de l'application des émulsions (E1) et (E2), et au terme de la durée de 28 jours de l'expérimentation, mesure le tour de poitrine (Mtp) et le tour de dessous de poitrine (Mdp) à l'aide d'un ruban centimétrique gradué.
On effectue en parallèle le contrôle de la courbe de poids pour chaque sujet (i) de chaque groupe afin de retirer lors de l'interprétation les données mesurées relatives aux sujets ayant subit une perte ou une prise de poids trop importante (évolution du poids du sujet de $\pm$ 3 kg par rapport au poids mesuré avant le début de l'étude). On note (j) le nombre de sujets dont l'évolution du poids est supérieure à $\pm$ 3 kg du poids initial mesuré avant le début de l'expérimentation. On calcule alors la population N de sujets pour chaque groupe qui sera la population pour laquelle les mesures seront interprétées selon la formule : N = 20 -j
La mesure centimétrique du tour de poitrine est réalisée sur chaque membre (i) des groupes (I) et (II) en prenant comme base les mamelons des deux seins de chaque sujet. Pour chaque membre (i) de chaque groupe, on mesure ainsi le tour de poitrine avant le début de l'expérimentation (Mpt0i) et le tour de poitrine à l'issue de la durée de l'expérimentation de 28 jours (Mtp28i). On calcule alors pour chaque membre de chaque groupe l'évolution du tour de poitrine $\Delta$Mpti selon la formule suivante :

$$\Delta Mpti = Mtp28i - Mpt0i$$

La moyenne arithmétique de l'évolution du tour de poitrine ($\Delta$Mptm) est ensuite calculée pour chaque groupe selon la formule suivante :

$$\Delta Mptm = [\textstyle\sum (\Delta Mpti)] / N$$

La mesure centimétrique du tour de dessous de poitrine est également réalisée sur chaque membre (i) des groupes (I) et (II) en prenant comme base le dessous des deux seins de chaque sujet. Pour chaque membre (i) de chaque groupe, on mesure ainsi le tour du dessous de poitrine avant le début de l'expérimentation (Mdp0i) et le tour du dessous de poitrine à l'issue de la durée de l'expérimentation de 28 jours (Mdp28i). On calcule alors pour chaque membre de chaque groupe l'évolution du tour de poitrine $\Delta$Mdpi selon la formule suivante :

$$\Delta Mdpt = Mdp28i - Mdp0i$$

La moyenne arithmétique de l'évolution du tour du dessous de poitrine ($\Delta$Mdpm) est ensuite calculée pour chaque groupe selon la formule suivante :

$$\Delta Mdpm = [\sum (\Delta Mdpi)] / N$$

Résultats obtenus

Les résultats des moyennes arithmétiques ∆Mptm et ∆Mdpm sont consignés dans le Tableau 6 ci-dessous pour les groupes (I) et (II).

Tableau 6

|  | Groupe (I) | Groupe (II) |
|---|---|---|
| ∆Mptm (cm) | +0,6 | 0 |
| ∆Mdpm (cm) | - 0,1 | 0 |

La moyenne arithmétique de l'évolution du tour de poitrine (∆Mptm) des membres du groupe (I), qui ont appliqué sur leurs seins l'émulsion huile-dans-eau (E1) comprenant le N-hexadécanoyl isoleucine a été mesurée à + 0,6 centimètre, pour une évolution du tour du dessous de poitrine (∆Mdpm) de - 0,1 centimètre.

La moyenne arithmétique de l'évolution du tour de poitrine (∆Mptm) des membres du groupe (II), qui ont appliqué sur leurs seins l'émulsion huile-dans-eau (E2) ne comprenant pas le N-hexadécanoyl isoleucine, a été mesurée à 0 centimètre, pour une évolution du tour du dessous de poitrine (∆Mdpm) de 0 centimètre.

Interprétation

On considère comme significative, une augmentation du tour de poitrine dont l'ensemble des valeurs (Mtp28i) sont statistiquement différentes des valeurs (Mtp0i).

On considère comme significative, une augmentation du tour de dessous de poitrine dont l'ensemble des valeurs (Mdp28i) sont statistiquement différentes des valeurs (Mdp0i).

L'analyse statistique utilisée pour évaluer la significativité de l'augmentation du tour de poitrine et de l'augmentation du tour de dessous de poitrine est réalisée par un test de Student bilatéral à variance inégale. Ainsi, l'ensemble des valeurs (Mtp28i) et des valeurs (Mdp28i) sont respectivement comparées à l'ensemble des valeurs (Mtp0i) et des valeurs (Mdp0i). Un seuil d'erreur(p) est fixé à 5%. Si p est inférieur ou égal à 0,05, l'ensemble des valeurs (Mtp28i) et des valeurs (Mdp28i) sont considérées comme significativement différentes respectivement de l'ensemble des valeurs (Mtp0i) et des valeurs (Mdp0i). Par conséquent, on peut conclure à une augmentation significative du tour de poitrine et du tour de dessous de poitrine. Si p est strictement supérieur à 0,05, l'ensemble des valeurs (Mtp28i) et des valeurs (Mdp28i) sont ne seront pas considérées comme significativement différentes respectivement de l'ensemble des valeurs (Mtp0i) et des valeurs (Mdp0i). Par conséquent, on ne pourra pas conclure à une augmentation significative du tour de poitrine et du tour de dessous de poitrine.

De plus, on considère comme significative une augmentation du tour de poitrine lorsque la moyenne arithmétique (∆Mptm) est supérieure ou égale à 0,2 cm, à condition que la moyenne arithmétique du tour du dessous de poitrine (∆Mdpm) ne soit pas supérieure à ± 0,2 cm.

Conclusion

On peut donc déduire des résultats que la présence du N-hexadécanoyl isoleucine dans l'émulsion huile-dans-eau (E1) a permis d'augmenter de façon significative le tour de poitrine moyen des membres du groupe (I) et a donc permis de procurer à ces sujets un effet «volumateur» sur leurs seins.

**b2 -** Evaluation de l'effet du N-hexadécanoyl isoleucine sur l'épaisseur de l'hypoderme du sein

Protocole

La mesure de l'épaisseur de l'hypoderme du sein par échographie de profondeur a été réalisée pour chaque membre de chaque groupe par l'intermédiaire d'un échographe Sonoline Antares™ commercialisé par la société SIEMENS muni d'une sonde de 6 cm de longueur et de 1 cm d'épaisseur. Avant chaque mesure, un gel de contact de marque Aquasonic100™ est appliqué sur le sein. La mesure est réalisée à une fréquence de 13 MHz par application directe de la sonde sur la peau du sein. La sonde est reliée à un logiciel de traitement des données mesurées et à une imprimante de type FUJI DryPix 7000™.

Pour chaque membre (i) de chaque groupe, cette évaluation est réalisée en effectuant les 3 mesures suivantes en 3 endroits différents du sein :

- Mesure de l'épaisseur de l'hypoderme est effectuée sur le côté latéral du sein (MLi),
- Mesure de l'épaisseur de l'hypoderme est effectuée sur la face supério-antérieure du sein (MSAi), et
- Mesure de l'épaisseur de l'hypoderme est effectuée sur la face infério-antérieure du sein (MIAi).

On effectue en parallèle le contrôle de la courbe de poids pour chaque sujet (i) de chaque groupe afin de retirer lors de l'interprétation les données mesurées relatives aux sujets ayant subit une perte ou une prise de poids trop importante (évolution du poids du sujet de $\pm$ 3 kg par rapport au poids mesuré avant le début de l'étude). On note (j) le nombre de sujets dont l'évolution du poids est supérieure à $\pm$ 3 kg du poids initial mesuré avant le début de l'expérimentation. On calcule alors la population N de sujets pour chaque groupe qui sera la population pour laquelle les mesures seront interprétées selon la formule : N = 20 - j

Pour chaque membre (i) de chaque groupe, on mesure ainsi :

- l'épaisseur de l'hypoderme sur le côté latéral du sein avant le début de l'expérimentation (ML0i) ;
- l'épaisseur de l'hypoderme sur le côté latéral du sein à l'issue de la durée de 28 jours de l'expérimentation (ML28i) ;
- l'épaisseur de l'hypoderme sur la face supério-antérieure du sein avant le début de l'expérimentation (MSA0i) ;
- l'épaisseur de l'hypoderme sur la face supério-antérieure du sein à l'issue de la durée de 28 jours de l'expérimentation (MSA28i) ;
- l'épaisseur de l'hypoderme sur la face inério-antérieure du sein avant le début de l'expérimentation (MIA0i) ; et
- l'épaisseur de l'hypoderme sur la face infério-antérieure du sein à l'issue de la durée de 28 jours de l'expérimentation (MIA28i).

Pour chaque membre de chaque groupe, on calcule ensuite :

- l'évolution de l'épaisseur de l'hypoderme sur le côté latéral du sein ($\Delta$MLi) selon la formule : $\Delta$MPLi = ML28i - ML0i ;
- l'évolution de l'épaisseur de l'hypoderme sur la face supério-antérieure du sein ($\Delta$MSAi) selon la formule : $\Delta$MSAi = MSA28i - MSA0i ; et
- l'évolution de l'épaisseur de l'hypoderme sur la face inério-antérieure du sein ($\Delta$MIAi) selon la formule $\Delta$MIAi = MIA28i - MIA0i.

Expression des résultats

A partir de la réalisation des mesures indiquées ci-dessus, on calcule ensuite pour chaque groupe :

- l'évolution moyenne de l'épaisseur de l'hypoderme sur le côté latéral du sein ($\Delta$MLm) selon la formule : $\Delta$MLm = [$\sum$ ($\Delta$MLi)] / N ;
- l'évolution moyenne de l'épaisseur de l'hypoderme sur la face supério-antérieure du sein ($\Delta$MSAm) selon la formule :

$$\Delta MSAm = [\textstyle\sum (\Delta MSAi)] / N \; ;$$

- l'évolution moyenne de l'épaisseur de l'hypoderme sur la face inério-antérieure du sein ($\Delta$MIAm) selon la formule :

$$\Delta MIAm = [\textstyle\sum (\Delta MIAi)] / N \; ;$$

- la moyenne de l'épaisseur de l'hypoderme sur le côté latéral du sein avant le début de l'expérimentation [Moy(ML0)] selon la formule :

$$[Moy(ML0)] = [\textstyle\sum (ML0i)] / N$$

- la moyenne de l'épaisseur de l'hypoderme sur la face supério-antérieure du sein avant le début de l'expérimentation [Moy(MSA0)] selon la formule :

$$[Moy(MSA0)] = [\sum (MSA0i)]/N$$

- la moyenne de l'épaisseur de l'hypoderme sur la face inério-antérieure du sein avant le début de l'expérimentation [Moy(MIA0)] selon la formule [Moy(MIA0)] = [$\sum$ (MIA0i)]/p
- le taux d'augmentation de l'épaisseur de l'hypoderme sur le côté latéral du sein (TML) selon la formule :

$$(TML) = [\Delta MLm / [Moy(ML0)]] \times 100$$

- le taux d'augmentation de l'épaisseur de l'hypoderme sur la face supério-antérieure du sein (TMSA) selon la formule :

$$(TMSA) = [\Delta MSAm / [Moy(MSA0)]] \times 100$$

- le taux d'augmentation de l'épaisseur de l'hypoderme sur la face infério-antérieure du sein (TMIA) selon la formule (TMIA) = [$\Delta$MIAm / [Moy(MIA0)]] x 100

Résultats obtenus

Les résultats des mesures réalisées pour les groupes (I) et (II), à savoir, les $\Delta$MLm, $\Delta$MSAm, $\Delta$MIAm, TML, TMSA et TMIA, sont consignés dans le Tableau 7 ci-dessous.

Tableau 7

|  | Groupe (I) | Groupe (II) |
|---|---|---|
| $\Delta$MLm (mm) | + 0,13 | + 0,04 |
| TML (%) | 1,0 | 0 |
| $\Delta$MSAm (mm) | + 1,14* | + 0,35* |
| TMSA (%) | 5,0 | 2,0 |
| $\Delta$MIAm (mm) | + 0,42 | - 1,19 |
| TMIA (%) | 1,0 | -4,0 |
| (*) : Résultats statistiquement significatifs par rapport à la valeur à t = 0 (test de Student bilatéral à variance inégale) avec p$\leq$ 0,05 | | |

Le taux d'augmentation de l'épaisseur de l'hypoderme sur la face supério-antérieure du sein (TMSA) des sujets du groupe (I), qui ont appliqué sur leurs seins l'émulsion huile-dans-eau (E1) comprenant le N-hexadécanoyl isoleucine, s'élève à 5% alors qu'une valeur de 2% de ce même taux d'augmentation de l'épaisseur de l'hypoderme sur la face supério-antérieure du sein (TMSA) a été calculée pour les sujets du groupe (II), qui ont appliqué sur leurs seins l'émulsion huile-dans-eau (E2) ne comprenant pas le N-hexadécanoyl isoleucine.

On observe aussi une augmentation moyenne de l'épaisseur de l'hypoderme sur la face supério-antérieure du sein ($\Delta$MSAm) de 1,14 mm pour les sujets du groupe (I) contre une valeur de 0,35 mm pour les sujets du groupe (II).

Cette augmentation significative de l'épaisseur de l'hypoderme sur la face supério-antérieure du sein pour les sujets du groupe (I) s'accompagne d'une stabilité de l'épaisseur de l'hypoderme sur la face inério-antérieure du sein ($\Delta$MIAm = + 0,42 mm) et d'une stabilité de l'épaisseur de l'hypoderme sur le côté latéral du sein ($\Delta$MLm = +0,13 mm).

Interprétation des résultats

On considère comme significative une augmentation moyenne de l'épaisseur de l'hypoderme sur le côté latéral du sein lorsque l'ensemble des valeurs (ML28i) sont statistiquement différentes des valeurs (ML0i).

On considère comme significative une augmentation moyenne de l'épaisseur de l'hypoderme sur la face supério-antérieure du sein lorsque l'ensemble des valeurs (MSA28i) sont statistiquement différentes des valeurs (MSA0i).

On considère comme significative une augmentation moyenne de l'épaisseur de l'hypoderme sur la sur la face inferio-antérieure du sein lorsque l'ensemble des valeurs (MIA28i) sont statistiquement différentes des valeurs (MIA0i).

L'analyse statistique utilisée pour évaluer la significativité de ces trois paramètres est un test de Student bilatéral à variance inégale. Ainsi, l'ensemble des valeurs (ML28i), (MSA28i), (MIA28i) sont respectivement comparées à l'ensemble des valeurs (ML0i), (MSA0i) et (MIA0i). Un seuil d'erreur (p) est fixé à 5%.

Si p est inférieur ou égal à 0,05, l'ensemble des valeurs (ML28i), (MSA28i), (MIA28i) sont considérées comme significativement différentes respectivement de l'ensemble des valeurs (ML0i), (MSA0i) et (MIA0i). Par conséquent, on peut conclure à une augmentation significative de l'épaisseur de l'hypoderme sur le côté latéral du sein et/ou sur la face supério-antérieure du sein et/ou sur la face inférieure-antérieure du sein.

Si p est strictement supérieur à 0,05, l'ensemble des valeurs (ML28i), (MSA28i), (MIA28i) ne seront pas considérées comme significativement différentes respectivement de l'ensemble des valeurs (ML0i), (MSA0i) et (MIA0i). Par conséquent, on ne pourra pas conclure à une augmentation significative de l'épaisseur de l'hypoderme sur le côté latéral du sein et/ou sur la face supério-antérieure du sein et/ou sur la face inférieure-antérieure du sein.

Conclusion

On peut donc déduire de ces résultats que la présence du N-hexadécanoyl isoleucine dans l'émulsion huile-dans-eau (E1) a permis d'augmenter de façon significative l'épaisseur de l'hypoderme sur la face supério-antérieure du sein sans que cette augmentation se réalise au détriment de l'épaisseur de l'hypoderme sur la face inférieure-antérieure du sein et/ou au détriment de l'épaisseur de l'hypoderme sur le côté latéral du sein. L'utilisation de N-hexadécanoyl isoleucine a donc permis de procurer à ces sujets un effet « repulpant » sur leurs seins.

**b3 -** Evaluation de l'effet du N-hexadécanoyl isoleucine sur l'augmentation du volume des seins à l'aide de la technique de projection de franges

Protocole

Ces évaluations ont été menées sur une sous-population aléatoire de 12 volontaires de la population utilisée pour les précédentes évaluations décrites dans les paragraphes b1) et b2) qui a été divisée en 2 groupes égaux de 6 volontaires (groupe III et groupe IV). Chaque membre du groupe (III) a appliqué sur ses 2 seins de façon biquotidienne et pendant une durée de 28 jours l'émulsion (E1) et chaque membre du groupe (IV) a appliqué sur ses 2 seins de façon biquotidienne et pendant une durée de 28 jours l'émulsion (E2).

Ces évaluations ont consisté à mesurer le volume des seins de chaque membre (i) de chaque groupe avant le début de l'expérimentation et à l'issue de la période de 28 jours suivant l'application des émulsions (E1) et (E2).

L'expérimentation a consisté à enregistrer une image en trois dimensions du sein de chaque membre (i) de chaque groupe, avant et après l'application pendant 28 jours des émulsions (E1) ou (E2), à l'aide d'un profilomètre à projection de franges d'interférences commercialisé sous l'appellation « 3D Primos Body™ ». Cet appareillage, se caractérisant par un champ de mesure de 300 mm x 200 mm, par une résolution latérale de 500 micromètres, par une résolution latérale supérieure ou égale à 30 micromètres et par une durée d'acquisition des données supérieure ou égale à 140 millisecondes, permet de projeter 2 faisceaux lumineux sur la surface à analyser et les images obtenues sont enregistrées par 2 caméras formant un angle de 20°. Un logiciel de calcul intégré au « 3D Primos Body™ » permet ensuite de calculer le volume de chaque zone présente sur l'image en 3 dimensions précédemment enregistrée.

Pour chaque membre (i) des groupes (III) et (IV) tels que précédemment définis, on calcule ensuite :

- le volume du sein concerné à partir de l'image en 3 dimensions enregistrée avant le début de l'expérimentation (V0i) ;
- le volume du sein concerné à partir de l'image en 3 dimensions enregistrée à l'issue de la durée de 28 jours de l'expérimentation (V28i) ;

pour chaque membre (i) des groupes (III) et (IV) tels que précédemment définis, on calcule ensuite l'évolution du volume du sein concerné $\Delta$Vi selon la formule :

$$\Delta V_i = (V28i) - (V0i)$$

Expression des résultats

A partir de la réalisation des mesures indiquées ci-dessus, on calcule ensuite pour chaque groupe :

- l'évolution moyenne du volume du sein (ΔVm) selon la formule

$$(\Delta Vm) = [\sum (\Delta Vi)] / 6 \; ;$$

- la moyenne du volume du sein avant le début de l'expérimentation [Moy(V0)] selon la formule :

$$[Moy(V0)] = [\sum (MV0i)] / 6$$

- le taux d'augmentation du volume du sein (TV) selon la formule :

$$(TV) = [(\Delta Vm) / [Moy(V0)]] \times 100$$

Résultats obtenus

Les résultats obtenus pour les groupes (III) et (IV), à savoir les ΔVm et TV sont consignés dans le Tableau 8 ci-dessous.

Tableau 8

|  | Groupe (III) | Groupe (IV) |
|---|---|---|
| (ΔVm) en $cm^3$ | +2,6* | -0,5* |
| (TV) en% | +1,0%* | 0%* |
| (*) : Résultats statistiquement significatifs par rapport à la valeur à t = 0 (test de Student bilatéral à variance inégale) avec p≤ 0,05 | | |

L'augmentation moyenne du volume du sein (ΔVm) des sujets du groupe (III), qui ont appliqué sur leurs seins l'émulsion huile-dans-eau (E1) comprenant le N-hexadécanoyl isoleucine, s'élève à 2,6 $cm^3$ contre une évolution de -0,5 $cm^3$, jugée comme non significative, pour les sujets du groupe (IV), qui ont appliqué sur leurs seins l'émulsion huile-dans-eau (E2) ne comprenant pas de N-hexadécanoyl isoleucine. De même, le taux d'augmentation du volume du sein (TV) a été calculée à +1,0% pour les sujets du groupe (III), qui ont appliqué sur leurs seins l'émulsion huile-dans-eau (E1) comprenant le N-hexadécanoyl isoleucine, contre une valeur de 0% pour les sujets du groupe (IV), qui ont appliqué sur leurs seins l'émulsion huile-dans-eau (E2) ne comprenant pas de N-hexadécanoyl isoleucine.

Interprétation des résultats

On considère comme significative une augmentation moyenne du volume du sein dont l'ensemble des valeurs (V28i) sont statistiquement différentes des valeurs (V0i). L'analyse statistique utilisée pour évaluer la significativité est un test de Student.
L'analyse statistique utilisée pour évaluer la significativité de l'augmentation du volume des seins est un test de Student bilatéral à variance inégale. Ainsi, l'ensemble des valeurs (V28i) sont comparées à l'ensemble des valeurs (V0i). Un seuil d'erreur (p) est fixé à 5%.
Si p est inférieur ou égal à 0,05, l'ensemble des valeurs (V28i) sont considérées comme significativement différentes de l'ensemble des valeurs (V0i). Par conséquent, on peut conclure à une augmentation significative du volume des seins. Si p est strictement supérieur à 0,05, l'ensemble des valeurs (V28i) ne seront pas considérées comme significativement différentes de l'ensemble des valeurs (V0i). Par conséquent, on ne pourra pas conclure à une augmentation significative du volume des seins.

Conclusion

On peut donc déduire de ces résultats que la présence du N-hexadécanoyl isoleucine dans l'émulsion huile-dans-eau (E1) a permis d'augmenter de façon significative le volume des seins des membres du groupe (III).

**b4 -** Conclusion générale

Les résultats expérimentaux mis en évidence dans les sections expérimentales b1), b2) et b3) mettent en évidence que l'application d'une composition cosmétique comprenant du N-hexadécanoyl isoleucine sur les seins permet, pour les sujets concernés, une augmentation significative du tour de poitrine, de l'épaisseur de l'hypoderme sur la face supério-antérieure du sein, et du volume des seins. En effet l'utilisation du N-hexadécanoyl isoleucine et l'application sur les seins d'une composition cosmétique comprenant du N-hexadécanoyl isoleucine a permis de générer un effet «volumateur» et un effet «repulpant» sur les seins des sujets concernés.

**2.2 - Mise en évidence de l'amélioration de l'élasticité de la peau et de l'effet « repulpant » d'une composition cosmétique comprenant du N-hexadécanoyl isoleucine sur le visage de sujets à peau mature.**

**a) - Préparation d'une émulsion huile-dans-eau comprenant du N-hexadécanoyl isoleucine (E3) et d'une émulsion huile-dans-eau « placébo » (E4).**

Les émulsions (E3) et (E4) sont préparées de la manière suivantes :

- Etape 1) : les composants de la phase huileuse et le système émulsionnant sont introduits successivement dans un bêcher, à une température de 80°C et sont soumis à une agitation mécanique pendant une durée de 15 minutes, à l'aide d'un agitateur muni d'un mobile de type « ancre », à une vitesse de 80 tours/minute à une température de 80°C, de façon à former un mélange homogène.
- Etape 2) : le polymère épaississant est ajouté sur le mélange obtenu à l'issue de l'étape 1) à une température de 80°C ainsi que la phase aqueuse, préalablement préparée à température ambiante par mélange de ses composants. Le mélange résultant est ensuite soumis à une agitation par l'intermédiaire d'un émulseur de type rotor-stator, commercialisé par la société SILVERSON, pendant une durée de 4 minutes à une vitesse de 3000 tours/minute à une température de 80°C.
- Etape 3) : Le mélange obtenu à l'issue de l'étape 2) est refroidi pendant 10 minute à une température de 30°C, et maintenu sous agitation pendant la durée de refroidissement par l'intermédiaire d'un agitateur mécanique muni d'un mobile de type «ancre», à une vitesse de 80 tours/minute.
- Etape 4 : l'agent conservateur SEPICIDE™LD, l'agent conservateur Chlorphenesine et le parfum « Petite Fleur», sont ensuite ajoutés sur le mélange issu de l'étape 3) à une température de 30°C. Le pH est ensuite ajusté à une valeur de 6,0 par ajout de triéthanolamine à 50%.

Les émulsions huile-dans-eau (E3) et (E4) sont de compositions (massiques) suivantes :

Tableau 9

|  | (E3) | (E4) |
|---|---|---|
| **Phase huileuse:** | | |
| Squalane végétal | 7,00% | 7,00% |
| Polyisobutène | 13,00% | 13,00% |
| Lanol P[5] | 1,50% | 1,50% |
| N-hexadécanoyl isoleucine | 1,00% | 0% |
| **Polymères épaisssissants** | | |
| SEPIPLUS™ 400[6] | 0,80% | 0,80% |
| KELTROL™ CGT[7] | 0,15% | 0,15% |
| **Phase aqueuse** | | |
| - Eau | Qs 100% | Qs 100% |
| - Triéthanolamine à 50% | Qs pH = 6,0 | Qs pH = 6,0 |
| - Glycérine | 5,00% | 5,00% |
| **Système émulsionnant** | | |
| MONTANOV™ 202[3] | 2,00% | 2,00% |
| MONTANOV™ 82[8] | 1,00% | 1,00% |
| **Additifs** | | |
| - SEPICIDE™ LD[9] | 0,70% | 0,70% |

(suite)

| Additifs | | |
|---|---|---|
| - Parfum Petite Fleur | 0,10% | 0,10% |
| - Chlorphenesine[10] | 0,30% | 0,30% |

(5) : Monopalmitate d'éthylène glycol, commercialisé par la société SEPPIC (6) : Latex inverse auto-inversible de copolymères tel que ceux décrits dans WO 2005/040230 (Dénomination INCI : Polyacrylate-13 & Polyisobutène & Polysorbate 20), commercialisé par la société SEPPIC ;

(7) : Gomme xanthane commercialisée par la société CP KELCO ;

(8) : Composition auto-émulsionnable à base d'alcool cétylstéarylique et de cocoglucosides, commercialisée par la société SEPPIC ;

(9) : Phénoxyéthanol, agent conservateur commercialisé par la société SEPPIC ;

(10) : Agent conservateur

**b) - Evaluation de l'effet «repulpant» et de l'amélioration de l'élasticité de la peau des émulsions huile-dans-eau (E3) et (E4) sur le visage de sujets à peau mature**

Les évaluations de l'effet "repulpant" et de l'amélioration de l'élasticité de la peau des émulsions huile-dans-eau sur le visage de sujets à peau mature sont réalisées en mesurant les critères suivants :

- l'évolution du volume du visage au niveau de la pommette à l'aide du Primos Body™ ;
- propriétés biomécaniques de la peau du visage à l'aide du Ballistomètre™.

Les évaluations de l'évolution du volume du visage au niveau de la pommette et des propriétés biomécaniques de la peau du visage ont été effectuées sur une population de 14 volontaires de sexe féminin, d'âge moyen de 58 ans, présentant une peau relâchée et creusée au niveau du visage, qui a été divisée en 2 groupes égaux de 7 volontaires (groupe V et groupe VI). Chaque membre du groupe (V) a appliqué sur son visage de façon biquotidienne et pendant une durée de 56 jours l'émulsion (E3) et chaque membre du groupe (VI) a appliqué sur son visage de façon biquotidienne et pendant une durée de 56 jours l'émulsion (E4).

**b1 -** Evaluation de l'effet « repulpant » du N-hexadécanoyl isoleucine sur le visage au niveau de la pommette à l'aide de la technique de projection de franges (Primos Body™)

Protocole

Cette évaluation a consisté à mesurer le volume du visage de chaque membre (i) de chaque groupe avant le début de l'expérimentation et à l'issue de la période de 56 jours suivant l'application des émulsions (E3) et (E4).

L'expérimentation a consisté à enregistrer une image en trois dimensions du visage de chaque membre (i) de chaque groupe, avant et après l'application pendant 56 jours des émulsions (E3) ou (E4), à l'aide d'un profilomètre à projection de franges d'interférences commercialisé sous l'appellation « 3D Primos Body™ ». Cet appareillage, se caractérisant par un champ de mesure de 300 mm x 200 mm, par une résolution latérale de 500 micromètres, par une résolution latérale supérieure ou égale à 30 micromètres et par une durée d'acquisition des données supérieure ou égale à 140 millisecondes, permet de projeter 2 faisceaux lumineux sur la surface à analyser et les images obtenues sont enregistrées par 2 caméras formant un angle de 20°. Un logiciel de calcul intégré au « 3D Primos Body™ » permet ensuite de calculer le volume de chaque zone présente sur l'image en 3 dimensions précédemment enregistrée.

Pour chaque membre (i) des groupes (V) et (VI) tels que précédemment définis, on calcule ensuite :

- le volume des joues au niveau des pommettes à partir de l'image en 3 dimensions enregistrée avant le début de l'expérimentation ($W0i$) ;
- le volume des joues au niveau des pommettes à partir de l'image en 3 dimensions enregistrée à l'issue de la durée de 56 jours de l'expérimentation ($W56i$).

Expression des résultats

Pour chaque membre (i) des groupes (V) et (VI) tels que précédemment définis, on calcule ensuite :

- l'évolution du volume du sein concerné $\Delta Wi$ selon la formule :

$$\Delta Wi = (W56i) - (W0i)$$

- l'évolution moyenne du volume des joues au niveau des pommettes ($\Delta Wm$) selon la formule :

$$(\Delta Wm) = [\sum(\Delta Wi)] / 7$$

Résultats

Les résultats obtenus pour les groupes (V) et (VI), à savoir les ($\Delta Wm$), sont consignés dans le Tableau 10 ci-dessous.

Tableau 10

|  | Groupe (V) | Groupe (VI) |
|---|---|---|
| ($\Delta Wm$) en ml | + 0,784* | + 0,276 |
| (*) : résultats statistiquement significatifs par rapport à la valeur à t = 0 (test de Student bilatéral à variance inégale) avec p$\leq$ 0,05 | | |

L'augmentation moyenne du volume des joues au niveau des pommettes ($\Delta Wm$) des sujets du groupe (V), qui ont appliqué sur leurs joues l'émulsion huile-dans-eau (E3) comprenant le N-hexadécanoyl isoleucine, s'élève à 0,784 ml, contre une augmentation de + 0,276 ml, considérée comme non statistiquement significative, pour les sujets du groupe (VI), qui ont appliqué sur leurs joues l'émulsion huile-dans-eau (E4) ne comprenant pas de N-hexadécanoyl isoleucine.

Interprétation des résultats

On considère comme significative et caractéristique d'un effet repulpant une augmentation moyenne du volume des joues au niveau des pommettes dont l'ensemble des valeurs (W56i) sont statistiquement différentes des valeurs (W0i).

L'analyse statistique utilisée pour évaluer la significativité est un test de Student à variance inégale. Si p est inférieur ou égal à 0,05, l'ensemble des valeurs (W56i) sont considérées comme significativement différentes de l'ensemble des valeurs (W0i). Par conséquent, on peut conclure à une augmentation significative du volume des joues au niveau des pommettes. Si p est strictement supérieur à 0,05, l'ensemble des valeurs (W56i) ne seront pas considérées comme significativement différentes de l'ensemble des valeurs (W0i). Par conséquent, on ne pourra pas conclure à une augmentation significative du volume des joues au niveau des pommettes.

Conclusion

On peut donc déduire de ces résultats que la présence du N-hexadécanoyl isoleucine dans l'émulsion huile-dans-eau (E3) a permis d'augmenter de façon significative le volume des joues au niveau des pommettes des membres du groupe (V) et donc d'induire un effet « repulpant » significatif sur le visage de ces sujets du groupe (V).

**b2 -** Evaluation de l'effet positif sur l'élasticité de la peau du N-hexadécanoyl isoleucine sur la peau du visage au niveau de la pommette à l'aide du Ballistomètre™,

Protocole

L'étude des propriétés biomécaniques de la peau permet de déterminer les effets assouplissants et/ou tenseurs de compositions après leur application sur la peau.

Les techniques existantes reposent sur l'étude de l'évolution des propriétés biomécaniques faisant suite à une action d'aspiration (utilisation d'un Cutometer™), de torsion (Dermal Torque Meter™), d'extension (Extensiomètre) de la peau ou au rebond d'une petite balle (Ballistomètre™) sur la peau.. Ces techniques permettent d'obtenir des résultats fiables, rapides, précises et mettent en oeuvre des protocoles non traumatisants pour la peau du sujet.

La peau possède 2 caractéristiques rhéologiques :

- une caractéristique élastique ;
- une caractéristique de tension qui varie selon les différentes zones du corps humain.

Les propriétés rhéologiques de la peau reposent sur sa structure qui consiste essentiellement en un réseau tridimensionnel de collagène et de fibres élastines.

Pour évaluer l'effet du N-hexadécanoyl isoleucine sur l'évolution des propriétés biomécaniques de la peau du visage au niveau de la pommette, la mise en oeuvre d'une mesure utilisant un Ballistomètre™ a été choisie. Le Ballistomètre™ permet d'illustrer l'évolution de la composante élastique de la peau.

L'évaluation menée dans le cadre de la présente étude a été réalisée avec un Ballistomètre™ BLS 780 commercialisé par la société DIA-STRON.

Le Ballistomètre™ BLS 780 consiste en une sonde manuelle, reliée à une unité de contrôle munie d'un logiciel de recueillement des données. La sonde manuelle contient un bras rigide en alliage d'aluminium, qui est monté sur un axe de torsion, et sur le bout dudit bras rigide se trouve une sphère vibrante d'un diamètre de 2 mm.

Lorsque la sphère vibrante de la sonde est mise au contact de la surface de la peau testée, une force de résistance est émise par la surface de la peau testée, qui induit un mouvement oscillatoire sur le ressort de l'axe de torsion. Ce mouvement oscillatoire est alors enregistré par l'unité de contrôle et traitée par le logiciel associé. Ce dispositif permet donc d'enregistrer les rebonds et leurs amortissements, ainsi que l'énergie associée, obtenus après application de la sphère vibrante sur la surface de la peau testée.

Le logiciel associé à l'unité centrale permet alors de calculer différents paramètres dont le « paramètre alpha » qui illustre le taux d'atténuation de l'énergie mécanique. Une valeur élevée de ce « paramètre alpha » caractérise une forte élasticité de la surface de la peau testée. Une augmentation au cours du temps du « paramètre alpha » témoigne d'un raffermissement de la peau.

Dans l'expérimentation objet de la présente étude, les <u>propriétés biomécaniques de la peau</u> sont évaluées au niveau de la pommette de la joue en appliquant la sonde du Ballistomètre™ sur ladite zone de la pommette de la joue avant et après l'application pendant 56 jours des émulsions (E3) ou (E4). Suite à cette application, pour chaque membre (i) des groupes (V) et (VI) tels que précédemment définis, on mesure ensuite :

- le « paramètre alpha » de la peau des joues au niveau des pommettes avant le début de l'expérimentation ($\alpha 0i$),
- le « paramètre alpha » de la peau des joues au niveau des pommettes à l'issue de la durée de 56 jours de l'expérimentation ($\alpha 56i$).

<u>Expression des résultats</u>

Pour chaque membre (i) des groupes (V) et (VI) tels que précédemment définis, on calcule ensuite :

- L'évolution du « paramètre alpha » de la peau des joues au niveau des pommettes ($\Delta\alpha i$) selon la formule :

$$(\Delta\alpha i) = (\alpha 56i) - (\alpha 0i)$$

et pour chaque groupe :
- L'évolution moyenne du « paramètre alpha » de la peau des joues au niveau des pommettes ($\Delta\alpha m$) selon la formule :

$$(\Delta\alpha m) = [\textstyle\sum (\Delta\alpha i)] / 7$$

- La moyenne du « paramètre alpha » de la peau des joues au niveau des pommettes avant le début de l'expérimentation [Moy($\alpha 0$)] selon la formule :

$$[\text{Moy}(\alpha 0)] = [\textstyle\sum (M\alpha 0i)]/7$$

- le taux d'évolution du « paramètre alpha » de la peau des joues au niveau des pommettes (T$\alpha$) selon la formule :

$$(T\alpha) = (\Delta\alpha m) / [\text{Moy}(\alpha 0)] \times 100$$

Résultats obtenus

Les résultats obtenus pour les groupes (V) et (VI), à savoir les évolutions moyennes du « paramètre alpha » de la peau des joues au niveau des pommettes ($\Delta\alpha m$) et le taux d'évolution du « paramètre alpha » de la peau des joues au niveau des pommettes (T$\alpha$) sont consignées dans le Tableau 11 ci-dessous.

**Tableau 11**

|  | Groupe (V) | Groupe (VI) |
|---|---|---|
| ($\Delta\alpha m$) | - 0,005* | 0,0 |
| (T$\alpha$) en% | - 10,0%* | + 1,0% |
| (*) : Résultats statistiquement significatifs par rapport à la valeur à t = 0 (test de Student bilatéral à variance inégale) avec p$\leq$ 0,05 | | |

L'évolution moyenne du « paramètre alpha » de la peau des joues au niveau des pommettes ($\Delta\alpha m$) des sujets du groupe (V), qui ont appliqué sur leurs joues l'émulsion huile-dans-eau (E3) comprenant le N-hexadécanoyl isoleucine, est de - 0,005, contre aucune évolution (($\Delta\alpha m$) = 0) pour les sujets du groupe (VI), qui ont appliqué sur leurs joues l'émulsion huile-dans-eau (E4) ne comprenant pas de N-hexadécanoyl isoleucine.
De même, le taux d'évolution du « paramètre alpha » de la peau des joues au niveau des pommettes (T$\alpha$) a été évalué à -10% pour les sujets du groupe (V), contre +1,0% pour les sujets du groupe (VI).

Interprétation des résultats

On considère comme significative et caractéristique d'une amélioration de l'élasticité de la peau des joues au niveau des pommettes une augmentation moyenne du « paramètre alpha » dont l'ensemble des valeurs ($\alpha 56i$) sont statistiquement différentes des valeurs ($\alpha 0i$). L'analyse statistique utilisée pour évaluer la significativité est un test de Student à variance inégale. Si p est inférieur ou égal à 0,05, l'ensemble des valeurs ($\alpha 56i$) sont considérées comme significativement différentes de l'ensemble des valeurs ($\alpha 0i$). Par conséquent, on peut conclure à une augmentation significative de l'élasticité de la peau des joues au niveau des pommettes. Si p est strictement supérieur à 0,05, l'ensemble des valeurs ($\alpha 56i$) ne seront pas considérées comme significativement différentes de l'ensemble des valeurs ($\alpha 0i$). Par conséquent, on ne pourra pas conclure à une augmentation significative de l'élasticité de la peau des joues au niveau des pommettes.

Conclusion

On peut donc déduire de ces résultats que la présence du N-hexadécanoyl isoleucine dans l'émulsion huile-dans-eau (E3) a permis d'induire de façon significative une amélioration de l'élasticité de la peau des joues au niveau des pommettes des membres du groupe (V).

**b3 -** Conclusion générale

Les résultats expérimentaux mis en évidence dans les sections expérimentales b1) et b2) du présent chapitre 2.2, montrent que l'application d'une composition cosmétique comprenant du N-hexadécanoyl isoleucine sur le visage permet, pour les sujets concernés, d'induire un effet « repulpant » et une amélioration de l'élasticité de la peau des joues au niveau des pommettes de façon significative.

**Références bibliographiques citées dans la description:**

- Dumont et al.: Analysis of the implications of the adipose tissue in facial morphology, from a revue of the literature and dissections of 10 half-faces. Annales de chirurgie plastique esthétique. 2007;52:169-205.
- Fève et al.: La différenciation adipocytaire : tout un programme... M/S. 1998;14:848-57
- Gregoire et al.: Understanding adipocyte différenciation. Physiological reviews. 1998 July;78(3):783-809
- Guo et al.: Aging results in paradoxical susceptibility of fat cell progenitors to lipotoxicity. Am J Physiol. Endocrinol. Metab. 2006 Dec.:1-54
- Karagiannides et al.: Altered expression of C/EBP family members results in decreased adipogenesis with aging. Am J Physiol Regulatory Integrative Comp Physiol. 2001 Jan.;280:R1772-80
- Karagiannides et al.: Increased CUG triplet repeat-binding protein-1 predisposes to impaired adipogenesis with aging. J Biol Chem. 2006 Aug.;281(32):23025-33
- Kirkland et al.: Adipogenesis and aging: does aging make fat go MAD? Exp Gerontol. 2002 June;37(6):757-67
- Smith et al.: The adipocyte life cycle hypothesis. Clinical Science. 2006;110:1-9
- Yu et al.: Chronological changes in metabolism and functions of cultured adipocytes: a hypothesis for cell aging in mature adipocytes. Am J Physiol Endocrinol Metab. 2004 March;286:402-410

**Revendications**

1. Utilisation cosmétique de N-hexadécanoyl isoleucine de formule (I) :

$$CH_3\text{-}(CH_2)_{14}\text{-}C(=O)\text{-}NH\text{-}CH(COOH)\text{-}CH(CH_3)\text{-}CH_2\text{-}CH_3 \qquad (I)$$

comme agent « volumateur » et/ou comme agent « repulpant » de la peau humaine.

2. Procédé de traitement cosmétique pour obtenir un effet « volumateur » et/ou « repulpant » de la peau d'une partie du corps humain choisie parmi les seins, le visage, les joues, les fesses et les paupières **caractérisé en ce qu'**il consiste à appliquer sur ladite partie une composition cosmétique comprenant une quantité efficace de N-hexadécanoyl isoleucine.

**Patentansprüche**

1. Kosmetische Verwendung von N-Hexadecanoylisoleucin der Formel (I):

$$CH_3\text{-}(CH_2)_{14}\text{-}C(=O)\text{-}NH\text{-}CH(COOH)\text{-}CH(CH_3)\text{-}CH_2\text{-}CH_3 \qquad (I)$$

als "Volumengeber"-Mittel und/oder als "Aufpolsterung"-Mittel für die menschliche Haut.

2. Verfahren zur kosmetischen Behandlung zum Erzielen eines "Volumengeber"- und/oder "Aufpolsterung"-Effekts auf der Haut eines Teils des menschlichen Körpers, der aus Brüsten, Gesicht, Wangen, Gesäß und Augenlidern ausgewählt ist, **dadurch gekennzeichnet, dass** es im Auftragen einer kosmetischen Zusammensetzung auf den genannten Teil besteht, die eine wirksame Menge N-Hexadecanoylisoleucin enthält.

**Claims**

1. Cosmetic use of N-hexadecanoyl isoleucine of formula (I):

$$CH_3\text{-}(CH_2)_{14}\text{-}C(=O)\text{-}NH\text{-}CH(COOH)\text{-}CH(CH_3)\text{-}CH_2\text{-}CH_3 \qquad (I)$$

as a "volumising" agent and/or as a "plumping" agent for human skin.

2. Cosmetic treatment process for achieving a "volumising" and/or "plumping" effect of the skin of a part of the human body chosen from the breasts, the face, the cheeks, the buttocks or the eyelids, **characterised in that** it consists in applying to said part a cosmetic composition comprising an effective amount of N-hexadecanoyl isoleucine.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008015639 A2
- WO 9014429 A1
- JP 2003096039 A
- WO 9809611 A
- FR 2873575 A1
- FR 2668080
- FR 2734496
- FR 2756195
- FR 2762317

- FR 2784680
- FR 2784904
- FR 2791565
- FR 2790977
- FR 2807435
- FR 2804432
- EP 0977626 A
- WO 2005040230 A

**Littérature non-brevet citée dans la description**

- **J. MORELLE.** Lipoaminoacides et cosmétologie. *Parfums cosmétiques savons de France,* 01 Février 1973, vol. 3 (2
- Analysis of the implications of the adipose tissue in facial morphology, from a revue of the literature and dissections of 10 half-faces. **DUMONT et al.** Annales de chirurgie plastique esthétique. 2007, vol. 52, 169-205
- **FÈVE et al.** *La différenciation adipocytaire : tout un programme... M/S.,* 1998, vol. 14, 848-57
- **GREGOIRE et al.** Understanding adipocyte différenciation. *Physiological reviews,* Juillet 1998, vol. 78 (3), 783-809
- **GUO et al.** Aging results in paradoxical susceptibility of fat cell progenitors to lipotoxicity. *Am J Physiol. Endocrinol. Metab.,* Décembre 2006, 1-54

- **KARAGIANNIDES et al.** Altered expression of C/EBP family members results in decreased adipogenesis with aging. *Am J Physiol Regulatory Integrative Comp Physiol.,* Janvier 2001, vol. 280, R1772-80
- **KARAGIANNIDES et al.** Increased CUG triplet repeat-binding protein-1 predisposes to impaired adipogenesis with aging. *J Biol Chem.,* Août 2006, vol. 281 (32), 23025-33
- **KIRKLAND et al.** Adipogenesis and aging: does aging make fat go MAD?. *Exp Gerontol.,* Juin 2002, vol. 37 (6), 757-67
- **SMITH et al.** The adipocyte life cycle hypothesis. *Clinical Science,* 2006, vol. 110, 1-9
- **YU et al.** Chronological changes in metabolism and functions of cultured adipocytes: a hypothesis for cell aging in mature adipocytes. *Am J Physiol Endocrinol Metab,* Mars 2004, vol. 286, 402-410